# EUROPEAN PATENT SPECIFICATION

(11) **EP 3 806 789 B1**
(45) Date of publication and mention of the grant of the patent: **25.10.2023**
(21) Application number: 19732898.2
(22) Date of filing: 14.06.2019
(51) Int. Cl.: A61F 13/00, A61B 5/00

(54) **ACCESSORY DEVICE OF A WOUND DRESSING SYSTEM, AND RELATED METHODS FOR COMMUNICATING OPERATING STATE**
ZUBEHÖRVORRICHTUNG EINES WUNDVERBANDSYSTEMS UND ZUGEHÖRIGE VERFAHREN ZUR ÜBERTRAGUNG DES BETRIEBSZUSTANDS
DISPOSITIF ACCESSOIRE D'UN SYSTÈME DE PANSEMENT ET PROCÉDÉS ASSOCIÉS POUR COMMUNIQUER UN ÉTAT DE FONCTIONNEMENT

(30) Priority: 15.06.2018 DK PA201870402
(43) Date of publication of application: 21.04.2021
(73) Proprietor: Coloplast A/S, 3050 Humlebaek (DK)
(72) Inventor: HANSEN, Jais Ask, 3630 Jaegerspris (DK); HVID, Niels, 2950 Vedbaek (DK); DAUGAARD-JENSEN, Daniel Gewecke, 2450 Copenhagen SV (DK); SOERENSEN, Peter Flintholm, 8000 Aarhus C (DK); ANDERSEN, Dan Boegsted, 2100 Copenhagen OE (DK)
(86) International application number: PCT/DK2019/050194
(87) International publication number: WO 2019/238198

(56) References cited:
- WO-A1-2018/115461
- WO-A1-2019/120434
- US-A1- 2014 058 344
- US-A1- 2014 298 928

## Description

The present disclosure relates to an accessory device of a wound dressing system and related methods for monitoring a wound dressing. wound dressings and in particular to wound dressings with sensing capability to facilitate monitoring of the wound/wound dressing during use of the wound dressing. A wound dressing system, and devices of the wound dressing system are also disclosed. The wound dressing system comprises a wound dressing, an accessory device, and a monitor device. In particular, the present disclosure relates to monitoring and/or communicating the operating state of the wound dressing.

### Brief Description of the Drawing

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Fig. 1 illustrates an exemplary wound dressing system,
Fig. 2 illustrates an exemplary monitor device of the wound dressing system,
Fig. 3 is a proximal view of a first adhesive layer of a wound dressing,
Fig. 4 is a schematic cross-section of an exemplary wound dressing,
Fig. 5 is a proximal view of an exemplary electrode assembly,
Fig. 6 is more detailed proximal view of a part of exemplary electrode assembly of Fig. 5,
Fig. 7 is a schematic cross-section of an exemplary wound dressing,
Fig. 8 is a proximal view of an exemplary first support layer,
Fig. 9 is a proximal view of an exemplary first masking layer,
Fig. 10 is a proximal view of an exemplary second support layer,
Fig. 11 is a proximal view of an exemplary second masking layer,
Fig. 12 is a distal view of an exemplary second support layer,
Fig. 13 is a proximal view of an exemplary second masking layer,
Fig. 14 is a distal view of an exemplary first support layer,
Fig. 15 illustrates an exemplary method of communicating the operating state according to the present disclosure,
Fig. 16 illustrates an exemplary accessory device according to the present disclosure,
Fig. 17 illustrates an exemplary user interface for communicating the operating state according to the present disclosure, and
Fig. 18 illustrates an exemplary user interface for communicating the operating state according to the present disclosure.

### Background

US 2014/0298928 A1 relates to appurtenances to a wound dressing and systems for monitoring wound dressings.

US 2014/0058344 A1 relates to an integrated system for assessing wound exudates from the wound of a patient. The system may contain functionality to detect, process and report various wound parameters.

### Detailed Description

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

In the following, whenever referring to proximal side or surface of a layer, an element, a device or part of a device, the referral is to the skin-facing side or surface, when a user wears the wound dressing. Likewise, whenever referring to the distal side or surface of a layer, an element, a device or part of a device, the referral is to the side or surface facing away from the skin, when a user wears the wound dressing. In other words, the proximal side or surface is the side or surface closest to the user, when the wound dressing is fitted on a user and the distal side is the opposite side or surface - the side or surface furthest away from the user in use.

The axial direction is defined as the direction away from the skin surface of the user, when a user wears the wound dressing. Thus, the axial direction is generally perpendicular to the skin or abdominal surface of the user.

The radial direction is defined as perpendicular to the axial direction. In some sentences, the words "inner" and "outer" may be used. These qualifiers should generally be perceived with respect to the radial direction, such that a reference to an "outer" element means that the element is farther away from a centre portion of the wound dressing than an element referenced as "inner". In addition, "innermost" should be interpreted as the portion of a component forming a centre of the component and/or being adjacent to the centre of the component. In analogy, "outermost" should be interpreted as a portion of a component forming an outer edge or outer contour of a component and/or being adjacent to that outer edge or outer contour.

The use of the word "substantially" as a qualifier to certain features or effects in this disclosure is intended to simply mean that any deviations are within tolerances that would normally be expected by the skilled person in the relevant field.

The use of the word "generally" as a qualifier to certain features or effects in this disclosure is intended to simply mean - for a structural feature: that a majority or major portion of such feature exhibits the characteristic in question, and - for a functional feature or an effect: that a majority of outcomes involving the characteristic provide the effect, but that exceptionally outcomes do no provide the effect.

The present disclosure relates to a wound dressing system and devices thereof, such as a wound dressing, a monitor device, and optionally one or more accessory devices. Further, methods related to an accessory device of the wound dressing system and accessory devices thereof are disclosed. An accessory device (also referred to as an external device) may be a mobile phone or other handheld device. An accessory device may be a personal electronic device, e.g. a wearable, such as a watch or other wrist-worn electronic device. An accessory device may be a docking station. The docking station may be configured to electrically and/or mechanically couple the monitor device to the docking station. The docking station may be configured for charging the monitor device and/or configured for transferring data between the monitor device and the docking station. The wound dressing system may comprise a server device. The server device may be operated and/or controlled by the wound dressing system manufacturer and/or a service centre.

A wound dressing system comprising a wound dressing and/or a monitor device is disclosed, wherein the monitor device is a monitor device as described herein.

The present disclosure provides a wound dressing system and devices thereof, such as a wound dressing, a monitor device, and optionally one or more accessory devices which either alone or together facilitate reliable monitoring of the wound dressing and operating state thereof. Accordingly, the wound dressing system and devices thereof enable providing information to the user about the operating state of the wound dressing, and in turn optionally enable providing an indication to the user or a caretaker of the remaining time frame for replacing the wound dressing without experiencing leakage and/or to provide optimum wound healing conditions. Further, the wound dressing system and devices thereof either alone or together facilitate reliable determination of the nature, severity and rapidness of exudate or other liquids wetting the absorbent core layer of the wound dressing. Depending on the degree of wetting and/or nature of the pattern of wetting propagation in the absorbent core layer, the wound dressing system and devices thereof enable determination and provision of an indication to the user/caretaker of the internal state of the wound dressing.

A wound dressing is disclosed, the wound dressing comprising a top layer, a first adhesive layer with a proximal surface configured for attachment of the wound dressing to the skin surface of a user; an absorbent core layer; and an electrode assembly comprising a plurality of electrodes optionally arranged on a distal side of the absorbent core layer. The top layer is optionally on a distal side of the electrode assembly. The wound dressing may comprise a monitor interface.

It is an advantage of the present disclosure that an optimum or improved use of a wound dressing is enabled and facilitated. In particular, the present disclosure facilitates that a wound dressing is not changed too early (leading to increased costs and/or material waste) nor too late (leading to adhesive failure, leakage and/or unsatisfactory wound healing conditions). Accordingly, the user or a health care professional is able to monitor and plan the use of the wound dressing.

Further, determination of moisture or wetting pattern types and classification of operating states of the wound dressing is useful in helping to reduce the risk of a user experiencing leakage from a wound dressing and/or in helping reduce the risk of unsatisfactory wound healing conditions. The present disclosure provides a simple, efficient, and easy-to-use wound dressing system with a high degree of comfort for a user.

The wound dressing comprises a first adhesive layer having a proximal surface configured for attachment of the wound dressing to the skin surface of a user. The first adhesive layer may comprise or be made of a first composition. The first composition may comprise silicone. The first adhesive layer may comprise a support layer with an adhesive material made of a first composition molded onto or otherwise attached to the support layer. The first composition may be a thermoset, curable adhesive material. An example of such adhesive material may be a silicone based adhesive material. The first composition may be a two-component system. Preferably, the first composition contains no solvent. Preferred first compositions include polyurethane, acrylic, silicone or polyethylene or polypropylene oxide based cross-linking types, e.g. as described in WO 2005/032401. The first composition may be a hotmelt type, which initially is heated to flow and subsequently cooled to gel or crosslink. Instead of curing upon cooling, the first composition may in some embodiments cure upon application of thermal energy.

The support layer of the first adhesive layer may be any suitable layer being water impermeable but vapour permeable. A suitable support layer may be a polyurethane film.

The first adhesive layer may have perforations or through-going openings arranged within an absorbing region, e.g. for allowing exudate from the wound to pass or flow through the perforations of the first adhesive layer to be absorbed by absorbent core layer arranged on the distal side of the first adhesive layer.

The perforations of the first adhesive layer may be made by punching, cutting or by applying high frequency mechanical vibrations, for example as disclosed in WO 2010/061228. The perforations may be arranged in a regular or random array, typically separated by 0.5 mm to 10 mm. The number of holes per cm² may be between 1 and 100, such as between 1 and 50 or even between 2 and 20. The perforations of the first adhesive layer may have a diameter in the range from 0.5 mm to 10 mm, such as in the range from 1 mm to 8 mm. In one or more exemplary wound dressings, the perforations of the first adhesive layer have a diameter in the range from 1 mm to 5 mm, e.g. from 1.5 mm to 5 mm, and even in the range from 2 mm to 4 mm.

The wound dressing comprises an absorbent core layer also denoted an absorbent pad. The absorbent core layer may be a uniform material or it may be a composite, for example in the form of a layered construction comprising layers of different texture and properties. The absorbent core layer may comprise foam, cellulose, super absorbent particles and/or fibres. The absorbent core layer may comprise a layer of foam facing the wound.

The absorbent core layer may comprise a polyurethane foam. The absorbent core layer may comprise a super absorbing layer.

The absorbent core layer and/or the first adhesive layer may contain active ingredients, such as ibuprofen, paracetamol, silver compounds or other medically active ingredients configured to reduce pain and/or to improve the healing of a wound. In one or more exemplary wound dressings, the absorbent core layer comprises a silver compound with antimicrobial properties.

The wound dressing comprises a top layer also denoted a backing layer. The top layer may be any suitable layer being water impermeable but vapour permeable. A suitable top layer may be a polyurethane film. The top layer is a protective layer protecting the absorbent core layer and other parts of the wound dressing from external strains and stress when the user wears the wound dressing. The electrodes, e.g. some or all the electrodes, may be arranged between the first adhesive layer and the top layer. The top layer may have a thickness in the range from 0.01 mm to 1.0 mm, e.g. in the range from 0.02 mm to 0.2 mm, such as 0.04 mm.

The top layer may comprise a top layer opening configured to allow for connection between the plurality of electrodes of the electrode assembly and terminals of a monitor device coupled to the wound dressing. For example, the top layer opening may allow for a first part of the electrode assembly to extend through the top layer to form a part of the monitor interface of the wound dressing.

The wound dressing may comprise a release liner, such as a one-piece, two-piece or a three-piece release liner. The release liner is a protective layer that protects adhesive layer(s) during transport and storage and is peeled off by the user prior to applying the wound dressing on the skin.

The electrode assembly comprises a plurality of sensor points or sensor zones distributed along a distal surface or distal side of the absorbent core layer. During use of the wound dressing, exudate from the wound may wet the distal surface of the absorbent core layer and in turn short-circuit an electrode pair of respective sensor points. Thus, wetting of a sensor point can be detected by determining the resistance and/or other electronic characteristic, such as capacitance, between two electrodes (sensing parts) of the sensor point.

The plurality of electrodes optionally comprises a first set of first electrodes and optionally a second set of second electrodes, wherein a sensing part of a first electrode and a sensing part of the second electrode may form a sensor point. The electrodes are electrically conductive and may comprise one or more of metallic (e.g. silver, copper, gold, titanium, aluminium, stainless steel), ceramic (e.g. ITO), polymeric (e.g. PEDOT, PANI, PPy), and carbonaceous (e.g. carbon black, carbon nanotube, carbon fibre, graphene, graphite) materials. An electrode comprises a connection part, e.g. to form a terminal of the monitor interface or for connecting the respective electrode to other components and/or to interface terminals/terminal elements. An electrode may comprise one or more conductor parts and/or one or more sensing parts.

A set of electrodes, such as the first set of electrodes and/or the second set of electrodes, may comprise one or a plurality of electrodes. In one or more exemplary electrode assemblies, a set of electrodes, such as the first set of electrodes and/or the second set of electrodes, may comprise one, two, three, four, five, or more electrodes. In one or more exemplary electrode assemblies, a first electrode of the first set of first electrodes forms a part of a first sensor point and a second sensor point of the plurality of sensor points. In one or more exemplary electrode assemblies, a first electrode of the first set of first electrodes forms a part of at least two sensor points, such as at least three sensor points. In one or more exemplary electrode assemblies, each of at least two first electrodes of the first set of first electrodes forms a part of at least two sensor points, such as at least three sensor points.

The first set of first electrodes may comprise one, two, three, or more electrodes. In one or more exemplary electrode assemblies, the first set of first electrodes comprises at least three electrodes, such as at least five electrodes.

In one or more exemplary electrode assemblies, a second electrode of the second set of second electrodes forms a part of a first sensor point and a third sensor point of the plurality of sensor points. In one or more exemplary electrode assemblies, a second electrode of the second set of second electrodes forms a part of at least two sensor points, such as at least three sensor points. In one or more exemplary electrode assemblies, each of at least two second electrodes of the second set of second electrodes forms a part of at least two sensor points, such as at least three sensor points.

The second set of second electrodes may comprise one, two, three, or more electrodes. In one or more exemplary electrode assemblies, the second set of second electrodes comprises at least three second electrodes, such as at least five electrodes.

In one or more exemplary electrode assemblies, first electrode(s) of the first set of electrodes comprises three or more sensing parts, such as three, four, five or more sensing parts. In one or more exemplary electrode assemblies, a first primary electrode and a first secondary electrode of the first set of electrodes each comprises three or more sensing parts, such as three, four, five or more sensing parts. In one or more exemplary electrode assemblies, a first tertiary electrode of the first set of electrodes comprises three or more sensing parts, such as three, four, five or more sensing parts. In one or more exemplary electrode assemblies, a first quaternary electrode of the first set of electrodes comprises three or more sensing parts, such as three, four, five or more sensing parts. In one or more exemplary electrode assemblies, a first quinary electrode of the first set of electrodes comprises three or more sensing parts, such as three, four, five or more sensing parts.

In one or more exemplary electrode assemblies, second electrode(s) of the second set of electrodes comprises three or more sensing parts, such as three, four, five or more sensing parts. In one or more exemplary electrode assemblies, a second primary electrode and a second secondary electrode of the second set of electrodes each comprises three or more sensing parts, such as three, four, five or more sensing parts. In one or more exemplary electrode assemblies, a second tertiary electrode of the second set of electrodes comprises three or more sensing parts, such as three, four, five or more sensing parts. In one or more exemplary electrode assemblies, a second quaternary electrode of the second set of electrodes comprises three or more sensing parts, such as three, four, five or more sensing parts. In one or more exemplary electrode assemblies, a second quinary electrode of the second set of electrodes comprises three or more sensing parts, such as three, four, five or more sensing parts.

Sensing part(s) may be ring-shaped, annular-shaped, or loop-shaped.

A first set of first electrodes with N1 first electrodes E_1_1, E_1_2, ..., E_1_N1 and a second set of second electrodes with N2 second electrodes E_2_1, E_2_2, ..., E_2_N2 allow for an electrode assembly with N1 times N2 sensor points. In one or more exemplary wound dressings, the number N1 of first electrodes in the first set of first electrodes is in the range from 1 to 30, such as in the range from 3 to 25, or in the range from 4 to 20. The number N1 of first electrodes in the first set of first electrodes may be larger than 4, such as larger than 5 or even larger than 6. In one or more exemplary wound dressings, the number N2 of second electrodes in the second set of second electrodes is in the range from 1 to 30, such as in the range from 3 to 25, or in the range from 4 to 20. The number N2 of second electrodes in the second set of second electrodes may be larger than 4, such as larger than 5 or even larger than 6.

The electrode assembly comprises a plurality of sensor points optionally distributed along a distal surface of the absorbent core layer. In one or more exemplary electrode assemblies, the plurality of sensor points comprises at least nine sensor points. The plurality of sensor points may be arranged in a matrix configuration. The plurality of sensor points may comprise at least 20 sensor points, such as 25, 30, 36, 40, 49 or more sensor points. In one or more exemplary electrode assemblies, the number of sensor points may be in the range from 20 to 50. Two electrodes of the electrode assembly, e.g. a first electrode of the first set of electrodes and a second electrode of the second set of electrodes (or respective sensing points thereof), may form a sensor point. A first electrode E_1_1 and a second electrode E_2_1 may form a (first) sensor point SP_1_1 (first electrode pair). The first electrode E_1_1 and a second electrode E_2_2 may form a (second) sensor point SP_1_2 (second electrode pair). The first electrode E_1_1 and a second electrode E_2_3 may form a (third) sensor point SP_1_3 (third electrode pair). A first electrode E_1_2 and the second electrode E_2_1 may form a (fourth) sensor point SP_2_1 (fourth electrode pair). The first electrode E_1_2 and the second electrode E_2_2 may form a (fifth) sensor point SP_2_2 (fifth electrode pair). The first electrode E_1_2 and the second electrode E_2_3 may form a (sixth) sensor point SP_2_3 (sixth electrode pair). A first electrode E_1_3 and the second electrode E_2_1 may form a (seventh) sensor point SP_3_1 (seventh electrode pair). The first electrode E_1_3 and the second electrode E_2_2 may form a (eighth) sensor point SP_3_2 (eighth electrode pair). The first electrode E_1_3 and the second electrode E_2_3 may form a (ninth) sensor point SP_3_3 (ninth electrode pair).

A distance, such as a center-to-center distance, between two neighbouring sensor points may be in the range from 2 mm to 50 mm, such as about 25 mm or about 30 mm. In one or more exemplary electrode assemblies, a distance, such as a center-to-center distance, between two neighbouring sensor points is in the range from 3 mm to 20 mm, such as in the range from 4 mm to 15 mm, e.g. about 5 mm, 6 mm, 7 mm, 8 mm, 9 mm, or 10 mm.

The electrode assembly may comprise one or more support layers including a first support layer. The first support layer may have a plurality of sensor point openings, e.g. for allowing exudate to pass through the first support layer. A sensor point opening of the first support layer may form a part of one or more sensor points of the electrode assembly. In one or more exemplary electrode assemblies, sensing part(s) of a first electrode surrounds sensor point opening(s) of the first support layer.

The first set of electrodes may be printed or arranged on the first support layer. In one or more exemplary electrode assemblies, the first set of electrodes are printed on a proximal surface of the first support layer. The first set of electrodes may be printed on a distal surface of the first support layer.

The second set of electrodes may be printed or arranged on the first support layer. In one or more exemplary electrode assemblies, the second set of electrodes are printed on a proximal surface of the first support layer. The second set of electrodes may be printed on a distal surface of the first support layer. The second set of electrodes may be printed or arranged on a support layer surface different than the support layer surface on which the first set of electrodes is printed or arranged. Arranging the first set of electrodes and the second set of electrodes on different support layer surfaces allows for provision of a larger number of sensor points using a smaller number of electrodes.

The electrode assembly may comprise a second support layer. The second set of electrodes may be printed or arranged on the second support layer. In one or more exemplary electrode assemblies, the second set of electrodes are printed on a proximal surface of the second support layer. The second set of electrodes may be printed on a distal surface of the second support layer.

The second support layer may have a plurality of sensor point openings, e.g. for allowing exudate to pass through the second support layer. A sensor point opening of the second support layer may form a part of one or more sensor points of the electrode assembly. In one or more exemplary electrode assemblies, sensing part(s) of a second electrode surrounds sensor point opening(s) of the first support layer and/or sensor point opening(s) of the second support layer.

A support layer, such as the first support layer and/or the second support layer, may comprise polymeric (e.g. polyurethane, PTFE, PVDF) and/or ceramic (e.g. alumina, silica) materials. In one or more exemplary wound dressings, the first support layer and/or the second support layer is/are made of thermoplastic polyurethane (TPU). The support layer material may be made of or comprise one or more of polyester, a thermoplastic elastomer (TPE), polyamide, polyimide, Ethylene-vinyl acetate (EVA), polyurea, and silicones. Exemplary thermoplastic elastomers of the first support layer and/or the second support layer are styrenic block copolymers (TPS, TPE-s), thermoplastic polyolefin elastomers (TPO, TPE-o), thermoplastic Vulcanizates (TPV, TPE-v), thermoplastic polyurethanes (TPU), thermoplastic copolyester (TPC, TPE-E), and thermoplastic polyamides (TPA, TPE-A).

The electrode assembly may comprise a spacing layer between the first support layer and the second support layer. A spacing layer between the first support layer and the second support layer may reduce the risk of false positives when detecting and/or determining moisture/liquid patterns or distributions. The spacing layer may have a plurality of sensor point openings, e.g. for allowing exudate to pass through the spacing layer. The spacing layer may be made of an absorbent material. The spacing layer may have a thickness in the range from 1 µm to 1 mm, such as about 10 µm or 100 µm.

The electrode assembly may have a first part and a second part. The first part may comprise at least some of or all the connection parts of the plurality of electrodes. The first part of the electrode assembly may extend through the top layer opening. In one or more exemplary wound dressings, at least some of the connection parts of the plurality of electrodes are arranged on a proximal surface of the electrode assembly. The first part of the electrode assembly may extend outside the absorbent core layer when seen in a radial direction. Accordingly, the connection parts of the plurality of electrodes may be arranged outside the absorbent core layer when seen in a radial direction. The first part may be elongated, e.g. having a length of at least 2 cm, such as at least 5 cm, e.g. in the range from 5 cm to 30 cm. An elongated first part may facilitate attachment and detachment of the monitor device and/or reduce the wear discomfort by allowing positioning of the monitor device away from the wound.

The electrode assembly may comprise a reinforcement element. The reinforcement element may form at least part of the first part of the electrode assembly. The reinforcement element optionally comprises a plurality of conductive paths respectively connected to connection parts of the plurality of electrodes. The conductive paths of the reinforcement element may form terminals of the monitor interface. The reinforcement element may be a flex circuit.

The second part may be arranged between the absorbent core layer and the top layer, i.e. the second part may be arranged to overlap with the absorbent core layer when seen in an axial direction. Thus, the second part of the electrode assembly may comprise the plurality of sensor points distributed along a distal surface of the absorbent core layer.

The electrode assembly/wound dressing may comprise one or more masking layers including a first masking layer isolating electrode parts of the plurality of electrodes. The first masking layer may isolate electrode parts of first electrodes of the first set of first electrodes.

The first masking layer may be printed on the first support layer and optionally cover one or more parts of electrodes printed or arranged on the first support layer. In one or more exemplary electrode assemblies, the first masking layer is printed on the proximal side of the first support layer and covering one or more parts of first electrodes and/or second electrodes. In one or more exemplary electrode assemblies, the first masking layer is printed on the distal side of the first support layer and covering one or more parts of first electrodes and/or second electrodes.

The first masking layer may be printed on the second support layer and optionally cover one or more parts of electrodes printed or arranged on the second support layer. In one or more exemplary electrode assemblies, the first masking layer is printed on the proximal side of the second support layer and covering one or more parts of second electrodes. In one or more exemplary electrode assemblies, the first masking layer is printed on the distal side of the second support layer and covering one or more parts of second electrodes.

The first masking layer may be divided in a plurality of first masking layer parts. The first masking layer may be arranged between the absorbent core layer and at least parts of the first electrodes to electrically isolate the parts of the first electrodes from the absorbent core layer. The first masking layer may be arranged between the absorbent core layer and at least parts of the second electrodes to electrically isolate the parts of the second electrodes from the absorbent core layer.

The first masking layer may comprise one or more, such as a plurality of, sensor point openings. A sensor point opening of the first masking layer optionally overlaps at least one electrode of the electrode assembly when seen in the axial direction, e.g. to form a sensor point. For example, a sensor point opening of the first masking layer may overlap a (sensing) part of a first electrode of the first set of electrodes and a (sensing) part of a second electrode of the second set of electrodes.

The electrode assembly may comprise a second masking layer isolating electrode parts of the plurality of electrodes.

The second masking layer may be printed on the first support layer and optionally cover one or more parts of electrodes printed or arranged on the first support layer. In one or more exemplary electrode assemblies, the second masking layer is printed on the distal side of the first support layer and covering one or more parts of second electrodes.

The second masking layer may be printed on the second support layer and optionally cover one or more parts of electrodes printed or arranged on the second support layer. In one or more exemplary electrode assemblies, the second masking layer is printed or arranged on the proximal side of the second support layer and covering one or more parts of second electrodes. In one or more exemplary electrode assemblies, the second masking layer is printed or arranged on the distal side of the second support layer and covering one or more parts of second electrodes.

The second masking layer may be divided in a plurality of second masking layer parts. The second masking layer may be arranged between the absorbent core layer and at least parts of the second electrodes to electrically isolate the parts of the second electrodes from the absorbent core layer. The second masking layer may be arranged between the first support layer and at least parts of the second electrodes to electrically isolate the parts of the second electrodes from the first support layer. The second masking layer may be arranged between the spacing layer and at least parts of the second electrodes to electrically isolate the parts of the second electrodes from the spacing layer.

The second masking layer may comprise one or more, such as a plurality of, sensor point openings. A sensor point opening of the second masking layer optionally overlaps at least one electrode of the electrode assembly when seen in the axial direction, e.g. to form a sensor point. For example, a sensor point opening of the second masking layer may overlap a (sensing) part of a first electrode of the first set of electrodes and/or a (sensing) part of a second electrode of the second set of electrodes.

A masking layer, e.g. the first masking layer and/or the second masking layer, may comprise one or more, such as a plurality of, terminal openings. A terminal opening may overlap with one or more connection parts of electrodes. In one or more exemplary wound dressings, each terminal opening overlaps with a single connection part of an electrode. A masking layer, e.g. the first masking layer and/or the second masking layer, may comprise polymeric (e.g. polyurethane, PTFE, PVDF) and/or ceramic (e.g. alumina, silica) materials. In one or more exemplary wound dressings, the first masking layer and/or the second masking layer is/are made of or comprises thermoplastic polyurethane (TPU). In one or more exemplary wound dressings, the first masking layer and/or the second masking layer is made of or comprises polyester. The masking element material may be made of or comprise one or more of polyester, a thermoplastic elastomer (TPE), polyamide, polyimide, Ethylene-vinyl acetate (EVA), polyurea, and silicones.

Exemplary thermoplastic elastomers of the first masking layer and/or the second masking layer are styrenic block copolymers (TPS, TPE-s), thermoplastic polyolefin elastomers (TPO, TPE-o), thermoplastic Vulcanizates (TPV, TPE-v), thermoplastic polyurethanes (TPU), thermoplastic copolyester (TPC, TPE-E), and thermoplastic polyamides (TPA, TPE-A).

The wound dressing comprises a monitor interface. The monitor interface may be configured for electrically and/or mechanically connecting the wound dressing (electrode assembly) to the monitor device. The monitor interface may be configured for wirelessly connecting the wound dressing to the monitor device. Thus, the monitor interface of the wound dressing is configured to electrically and/or mechanically couple the wound dressing and the monitor device.

The monitor interface of the wound dressing may comprise, e.g. as part of a first connector of the monitor interface, a coupling part for forming or configured for forming a mechanical connection, such as a releasable coupling between the monitor device and the wound dressing. The coupling part may be configured to engage with a coupling part of the monitor device for releasably coupling the monitor device to the wound dressing.

The coupling part may be attached, such as glued, welded, press-fitted, or soldered, to the electrode assembly. In one or more exemplary wound dressings, the coupling part extends through the top layer opening, i.e. a part of the coupling part may extend on the proximal side of the top layer and a part of the coupling part may extend on the distal side of the top layer. The coupling part may be attached such as glued, welded, press-fitted, or soldered, to the top layer.

In one or more exemplary wound dressings, the first part of the electrode assembly extends into the coupling part, i.e. the coupling part may accommodate at least a part of the first part of the electrode assembly.

The monitor interface of the wound dressing may comprise, e.g. as part of a first connector of the monitor interface, a plurality of terminals, such as two, three, four, five, six, seven, eight or more terminals, for forming or configured to form electrical connections with respective terminals of the monitor device. The monitor interface may comprise a ground terminal element forming a ground terminal. The monitor interface may comprise a first terminal element forming a first terminal, a second terminal element forming a second terminal and optionally a third terminal element forming a third terminal. The monitor interface may comprise a fourth terminal element forming a fourth terminal and/or a fifth terminal element forming a fifth terminal. The monitor interface optionally comprises a sixth terminal element forming a sixth terminal. The terminal elements of the monitor interface may contact respective electrodes (connection parts) of the wound dressing/electrode assembly. In one or more exemplary wound dressings, a first intermediate element may be arranged between the terminal elements and the first adhesive layer. The first intermediate element may cover or overlap terminal element(s) of the wound dressing when seen in the axial direction. Thus, the first adhesive layer may be protected or experience more evenly distributed mechanical stress from the terminal elements of the wound dressing, in turn reducing the risk of terminal elements penetrating or otherwise damaging the first adhesive layer. The first intermediate element may protect or mechanically and/or electrically shield the first adhesive layer from the terminal elements of the wound dressing.

A terminal element, such as the ground terminal element, the first terminal element, the second terminal element, the third terminal element, the fourth terminal element, the fifth terminal element and/or the sixth terminal element, may comprise a distal end and a proximal end. A terminal element, such as the ground terminal element, the first terminal element, the second terminal element, the third terminal element, the fourth terminal element, the fifth terminal element and/or the sixth terminal element, may comprise a distal part (with a distal end), a centre part, and/or a proximal part (with a proximal end). The centre part may be between the distal part and the proximal part. The proximal end/proximal part of a terminal element may contact a connection part of an electrode. A terminal element, such as the ground terminal element, the first terminal element, the second terminal element, the third terminal element, the fourth terminal element, the fifth terminal element and/or the sixth terminal element, may be gold plated copper.

In one or more exemplary wound dressings, connection parts of electrodes of the electrode assembly form respective terminals of the monitor interface.

The monitor device comprises a processor and one or more interfaces, such as a first interface and/or a second interface. The monitor device may comprise a memory for storing wound data and/or parameter data based on the wound data.

The first interface is connected to the processor and the memory. The first interface is configured for collecting wound data from the wound dressing coupled to the first interface. The wound data, also denoted WD, comprises wound data from sensor points of the wound dressing, e.g. first wound data WD_1 from a first sensor point, e.g. a first electrode pair, of the wound dressing, second wound data WD_2 from a second sensor point, e.g. a second electrode pair, of the wound dressing, and optionally third wound data WD_3 from a third sensor point, e.g. a third electrode pair, of the wound dressing. In one or more exemplary monitor devices, the wound data comprises wound data for each sensor point of the wound dressing. For example, for a wound dressing with N sensor points, the wound data WD may comprise WD_1, WD_2, ..., WD_N. The number N of sensor points of the wound dressing may be at least 9, such as at least 20 or even larger than 50.

The processor is configured to apply a processing scheme. To apply a processing scheme comprises to obtain parameter data based on the wound data, e.g. the first wound data WD_1, the second wound data WD_2, and the third wound data WD_3; and to determine an operating state of the wound dressing based on the parameter data. The parameter data may comprise one or more of first parameter data, also denoted P_1, based on the first wound data WD_1, second parameter data, also denoted P_2, based on the second wound data WD_2, and third parameter data, also denoted P_3, based on the third wound data WD_3.

The operating state of the wound dressing is optionally indicative of a degree of wetting of the absorbent core layer of the wound dressing. The operating state is optionally indicative of a degree of wetting of the distal surface of the absorbent core layer. The operating state may be indicative of a wetting pattern or wetting distribution on the distal surface or distal side of the absorbent core layer.

The monitor device is optionally configured to, in accordance with a determination that the operating state is a first operating state, transmit a first monitor signal comprising monitor data indicative of the first operating state of the wound dressing via the second interface. The first operating state of the wound dressing may correspond to a situation wherein the absorbent core layer is wetted to a first degree on the distal surface of the absorbent core layer and/or wherein a first wetting pattern is detected on the distal surface of the absorbent core layer.

The monitor device is optionally configured to, in accordance with a determination that the operating state is a second operating state, transmit a second monitor signal comprising monitor data indicative of the second operating state of the wound dressing via the second interface. The second operating state of the wound dressing may correspond to a situation wherein the absorbent core layer is wetted to a second degree (different from the first degree) on the distal surface of the absorbent core layer and/or wherein a second wetting pattern is detected on the distal surface of the absorbent core layer.

In one or more exemplary monitor devices, to determine an operating state of the wound dressing is based on a first criteria set based on first parameter data and/or second parameter data of the parameter data, wherein the operating state is determined to be the first operating state if the first criteria set is satisfied. The first criteria set optionally comprises a first primary criterion based on the first parameter data, and a first secondary criterion based on the second parameter data.

In one or more exemplary monitor devices, to determine an operating state of the wound dressing is based on a first threshold set comprising one or more first threshold values.

In one or more exemplary monitor devices, to determine an operating state of the wound dressing is based on a second criteria set based on first parameter data and second parameter data of the parameter data, wherein the operating state is determined to be the second operating state if the second criteria set is satisfied. The second criteria set optionally comprises a second primary criterion based on the first parameter data, and a second secondary criterion based on the second parameter data. Applying first and second criteria set based on first parameter data and second parameter data allows for a distinction between different degrees and/or patterns of wetting.

In one or more exemplary monitor devices, to determine an operating state of the wound dressing is based on a second threshold set comprising one or more second threshold values.

In one or more exemplary monitor devices, to determine an operating state of the wound dressing is based on a default criteria set based on the parameter data, wherein the operating state is determined to be the default operating state if the default criteria set is satisfied, and optionally in accordance with a determination that the operating state is the default operating state, transmit a default monitor signal comprising monitor data indicative of the default operating state of the wound dressing. The default operating state may correspond to no wetting or a low degree of wetting of the (distal surface or side of) absorbent core layer.

In one or more exemplary monitor devices, to determine an operating state of the wound dressing is based on a third criteria set based on third parameter data of the parameter data, wherein the operating state is determined to be the third operating state if the third criteria set is satisfied. The third operating state of the wound dressing may correspond to a situation wherein the absorbent core layer is wetted to a third degree on the distal surface of the absorbent core layer and/or wherein a third wetting pattern is detected on the distal surface of the absorbent core layer.

The monitor device is optionally configured to, in accordance with a determination that the operating state is the third operating state, transmit a third monitor signal comprising monitor data indicative of the third operating state of the wound dressing.

The parameter data may be indicative of resistance between the two electrodes of an electrode pair forming a sensor point. For example, the first parameter data, the second parameter data, and the third parameter data, may be indicative of resistance between first electrode pair of the first sensor point, second electrode pair of the second sensor point, and third electrode pair of the third sensor point, respectively. Wetting of the distal surface of the absorbent core layer with exudate, i.e. exudate from the wound being absorbed by the absorbent core layer, is detected by a reduced resistance between the two electrodes of the sensor point(s). The sensor points are arranged or distributed along the distal surface of the absorbent core layer allowing the monitor device to detect and/or derive a degree of wetting and/or a wetting pattern or wetting distribution on the distal surface of the of the absorbent core layer.

In one or more exemplary monitor devices, the parameter data are indicative of a rate of change in resistance between the two electrodes of an electrode pair forming a sensor point. The first parameter data, the second parameter data, and the third parameter data may be indicative of a rate of change in resistance between first electrode pair of the first sensor point, second electrode pair of the second sensor point, and third electrode pair of the third sensor point, respectively.

In one or more exemplary monitor devices, the wound data comprises fourth wound data from a fourth sensor point of the wound dressing, and wherein to apply a processing scheme comprises to obtain fourth parameter data based on the fourth wound data, and determine an operating state of the wound dressing based on the fourth parameter data.

The monitor device is optionally configured to, in accordance with a determination that the operating state is a fourth operating state, transmit a fourth monitor signal comprising monitor data indicative of the fourth operating state of the wound dressing. The fourth operating state of the wound dressing may correspond to a situation wherein the absorbent core layer is wetted to a fourth degree on the distal surface of the absorbent core layer and/or wherein a fourth wetting pattern is detected on the distal surface of the absorbent core layer.

In one or more exemplary monitor devices, to obtain parameter data comprises to obtain common parameter data of the parameter data based on a plurality of the first wound data, the second wound data, and the third wound data, and wherein to determine the operating state of the wound dressing is based on the common parameter data.

In one or more exemplary monitor devices, to determine an operating state comprises to determine a degree of wetting of the absorbent core layer, such as a degree of wetting of the distal side or surface of the absorbent core material. To determine a degree of wetting of the absorbent core material may comprising to determine if the degree of wetting satisfies first wetting criterion and/or if the degree of wetting satisfies second wetting criterion.

The monitor device is optionally configured to, in accordance with the degree of wetting satisfying first wetting criterion, setting the operating state to be the first operating state and optionally include the degree of wetting in the monitor data.

The monitor device is optionally configured to, in accordance with the degree of wetting satisfying a second wetting criterion, setting the operating state to be the second operating state and optionally include the degree of wetting in the monitor data.

In one or more exemplary monitor devices, to determine an operating state comprises to determine a wetting pattern of the absorbent core layer, such as a wetting pattern on the distal side or surface of the absorbent core material, and optionally to determine a pattern type of the wetting pattern from a plurality of pattern types. To determine a pattern type of the wetting pattern may comprise to determine if the wetting pattern satisfies first pattern type criterion, wherein the pattern type is determined as being a first pattern type if the first pattern type criterion is satisfied. To determine a pattern type of the wetting pattern may comprise to determine if the wetting pattern satisfies second pattern type criterion, wherein the pattern type is determined as being a second pattern type if the second pattern type criterion is satisfied.

The monitor device may be configured to, in accordance with the pattern type being a first pattern type, setting the operating state to be the first operating state and optionally including a pattern representation of the wetting pattern in the monitor data. The pattern representation may comprise a pattern type identifier and/or pattern data indicative of parameter data.

The monitor device may be configured to, in accordance with the pattern type being a second pattern type, setting the operating state to be the second operating state and optionally including a pattern representation of the wetting pattern in the monitor data. The pattern representation may comprise a pattern type identifier and/or pattern data indicative of parameter data.

The monitor device comprises a second interface connected to the processor. The second interface may comprise a loudspeaker connected to the processor, and wherein the processor is configured to transmit a monitor signal via the loudspeaker. In one or more exemplary monitor devices, the second interface comprises an antenna and a wireless transceiver, and wherein the processor is configured to transmit a monitor signal as a wireless monitor signal via the antenna and the wireless transceiver.

A wound dressing system is disclosed, the wound dressing system comprising a wound dressing and a monitor device, the wound dressing comprising an absorbent core layer, wherein the monitor device is a monitor device as described herein.

To obtain first parameter data based on the first wound data may comprise determining one or more first parameters based on the first wound data. To obtain second parameter data based on the second wound data may comprise determining one or more second parameters based on the second wound data. To obtain third parameter data based on the third wound data may comprise determining one or more third parameters based on the third wound data. In one or more exemplary monitor devices, determination of an operating state may be based on one or more first parameters, such as first primary parameter and/or first secondary parameter of first parameter data. In one or more exemplary monitor devices, determination of an operating state may be based on one or more second parameters, such as second primary parameter and/or second secondary parameter of the second parameter data. In one or more exemplary monitor devices, determination of an operating state may be based on one or more third parameters, such as third primary parameter and/or third secondary parameter of the third parameter data. In one or more exemplary monitor devices, determination of an operating state may be based on one or more fourth parameters, such as fourth primary parameter and/or fourth secondary parameter of the fourth parameter data.

Parameter data, P_1, P_2, ..., P_N, may comprise respective parameters p_1_1, p_2_1, ..., p_N_1 indicative of resistance between the respective two electrodes forming a sensor point of the wound dressing. Parameter data, P_1, P_2, ..., P_N, may comprise respective parameters p_1_2, p_2_2, ..., p_N_2 each indicative of a rate of change in resistance between the respective two electrodes forming a sensor point of the wound dressing. Accordingly, p_1_1 of P_1 may be the resistance measured between the first electrode E_1_1 and the second electrode E_2_1 forming the first sensor point SP_1_1, p_2_1 of P_2 may be the resistance measured between first electrode E_1_1 and the second electrode E_2_2 forming the second sensor point SP_1_2.

In one or more exemplary monitor devices and/or methods, to determine an operating state of the wound dressing is based on a first criteria set based on the first parameter data P_1 (p_1_1) and/or one or more other parameter data P_2 (p_2_1), P_3 (p_3_1), ..., P_N (p_N_1), wherein the operating state is determined to be the first operating state if the first criteria set is satisfied. The first criteria set may comprise one or more first criteria based on one or more of P_1, P_2, ..., P_N. The first criteria set may comprise a first primary criterion based on P_1 (p_1_1). The first criteria set may comprise a first secondary criterion based on P_2 (p_2_1). The first criteria set may comprise a first tertiary criterion based on P_3 (p_3_1). The first criteria set may comprise N first criteria respectively based on P_1, P_2, ..., P_N.

In one or more exemplary monitor devices and/or methods, to determine an operating state of the wound dressing is based on a second criteria set based on the second parameter data P_2 (p_2_1) and/or one or more other parameter data P_1 (p_1_1), P_3 (p_3_1), ..., P_N (p_N_1), wherein the operating state is determined to be the second operating state if the second criteria set is satisfied. The second criteria set may comprise one or more second criteria based on one or more of P_1, P_2, ..., P_N. The second criteria set may comprise a second primary criterion based on P_1 (p_1_1). The second criteria set may comprise a second secondary criterion based on P_2 (p_2_1). The second criteria set may comprise a second tertiary criterion based on P_3 (p_3_1). The second criteria set may comprise N second criteria respectively based on P_1, P_2, ..., P_N.

In one or more exemplary monitor devices and/or methods, to determine an operating state of the wound dressing may comprise to determine the number of parameters p_1_1, p_2_1, ..., p_N_1 having resistances less than a first threshold as a first common parameter of common parameter data. The operating state of the wound dressing may be based on the number of parameters p_1_1, p_2_1, ..., p_N_1 having resistances less than a first threshold. The operating state of the wound dressing may be determined as the first operating state if the first common parameter being the number of parameters p_1_1, p_2_1, ..., p_N_1 having resistances less than a first threshold is in a first range, e.g. from 0.25N to 0.5N. The first operating state may be indicative of a low-wetted absorbent core layer, i.e. a high degree of remaining absorbent capacity of the wound dressing/absorbent core layer. The operating state of the wound dressing may be determined as the second operating state if the first common parameter being the number of parameters p_1_1, p_2_1, ..., p_N_1 having resistances less than a first threshold is in a second range, e.g. from 0.5N to 0.75N. The second operating state may be indicative of a medium-wetted absorbent core layer, i.e. a medium degree of remaining absorbent capacity of the wound dressing/absorbent core layer.

The operating state of the wound dressing may be determined as a default operating state if the first common parameter being the number of parameters p_1_1, p_2_1, ..., p_N_1 having resistances less than a first threshold is less than a default threshold. The default threshold may be a fixed value or based on the number N of sensor points. The default threshold may be 0.1 N or 0.25 N. The processor is optionally configured to, in accordance with a determination that the operating state is the default operating state, transmit a default monitor signal comprising monitor data indicative of the default operating state of the wound dressing. The default operating state may be indicative of a substantially non-wetted absorbent core layer, i.e. a very high degree of remaining absorbent capacity of the wound dressing/absorbent core layer.

In one or more exemplary monitor devices and/or methods, to determine an operating state of the wound dressing is based on a default criteria set based on parameter data P_1, P_2, ..., P_N, wherein the operating state is determined to be the default operating state if the default criteria set is satisfied. The monitor device may be configured to, in accordance with a determination that the operating state is the default operating state, transmit a default monitor signal comprising monitor data indicative of the default operating state of the wound dressing.

In one or more exemplary monitor devices and/or methods, to determine an operating state of the wound dressing is based on a third criteria set based on parameter data P_1, P_2, ..., P_N, wherein the operating state is determined to be the third operating state if the third criteria set is satisfied. The operating state of the wound dressing may be determined as the third operating state if the first common parameter being the number of parameters p_1_1, p_2_1, ..., p_N_1 having resistances less than a first threshold is in a third range, e.g. from 0.75N to 0.9N.

The processor is optionally configured to, in accordance with a determination that the operating state is the third operating state, transmit a third monitor signal comprising monitor data indicative of the third operating state of the wound dressing. In one or more exemplary monitor devices, the third operating state of the wound dressing corresponds to a situation wherein the absorbent core layer is wetted to a third degree on the distal surface. The third degree may be indicative of low degree of remaining absorbent capacity of the wound dressing/absorbent core layer.

In one or more exemplary monitor devices and/or methods, to determine an operating state of the wound dressing is based on a fourth criteria set based on parameter data P_1, P_2, ..., P_N, wherein the operating state is determined to be the fourth operating state if the fourth criteria set is satisfied. The operating state of the wound dressing may be determined as the fourth operating state if the first common parameter being the number of parameters p_1_1, p_2_1, ..., p_N_1 having resistances less than a first threshold is in a fourth range, e.g. from 0.9N to N.

The processor is optionally configured to, in accordance with a determination that the operating state is the fourth operating state, transmit a fourth monitor signal comprising monitor data indicative of the fourth operating state of the wound dressing. In one or more exemplary monitor devices, the fourth operating state of the wound dressing corresponds to a situation wherein the absorbent core layer is wetted to a fourth degree on the distal surface. The fourth degree may be indicative of very low or no degree of remaining absorbent capacity of the wound dressing/absorbent core layer.

The monitor device comprises a monitor device housing optionally made of a plastic material. The monitor device housing may be an elongate housing having a first end and a second end. The monitor device housing may have a length or maximum extension along a longitudinal axis in the range from 1 cm to 15 cm. The monitor device housing may have a width or maximum extension perpendicular to the longitudinal axis in the range from 0.5 cm to 3 cm. The monitor device housing may be curve-shaped.

The monitor device comprises a first interface. The first interface may be configured as a wound dressing interface for electrically and/or mechanically connecting the monitor device to the wound dressing. Thus, the wound dressing interface is configured to electrically and/or mechanically couple the monitor device and the wound dressing. The first interface may be configured as an accessory device interface for electrically and//or mechanically connecting the monitor device to an accessory device. The first interface may be configured for coupling to a docking station of the wound dressing system, e.g. for charging the monitor device and/or for data transfer between the monitor device and the docking station.

The first interface of the monitor device may comprise a plurality of terminals, such as two, three, four, five, six, seven or more terminals, for forming electrical connections with respective terminals and/or electrodes of the wound dressing. One or more terminals of the first interface may be configured for forming electrical connections with an accessory device, e.g. with respective terminals of a docking station. The first interface may comprise a ground terminal. The first interface may comprise a first terminal, a second terminal and optionally a third terminal. The first interface may comprise a fourth terminal and/or a fifth terminal. The first interface optionally comprises a sixth terminal. In one or more exemplary monitor devices, the first interface has M terminals, wherein M is an integer in the range from 4 to 16.

The first interface of the monitor device may comprise a coupling part for forming a mechanical connection, such as a releasable coupling between the monitor device and the wound dressing. The coupling part and the terminals of the first interface form (at least part of) a first connector of the monitor device.

The monitor device comprises a power unit for powering the monitor device. The power unit may comprise a battery. The power unit may comprise charging circuitry connected to the battery and terminals of the first interface for charging the battery via the first interface, e.g. the first connector. The first interface may comprise separate charging terminal(s) for charging the battery.

The monitor device may comprise a sensor unit with one or more sensors. The sensor unit is connected to the processor for feeding sensor data to the processor. The sensor unit may comprise an accelerometer for sensing acceleration and provision of acceleration data to the processor. The sensor unit may comprise a temperature sensor for provision of temperature data to the processor.

The monitor device comprises a second interface connected to the processor. The second interface may be configured as an accessory interface for connecting, e.g. wirelessly connecting, the monitor device to one or more accessory devices and transmitting monitor data to the accessory device. The second interface may comprise an antenna and a wireless transceiver, e.g. configured for wireless communication at frequencies in the range from 2.4 to 2.5 GHz. The wireless transceiver may be a Bluetooth transceiver, i.e. the wireless transceiver may be configured for wireless communication according to Bluetooth protocol, e.g. Bluetooth Low Energy, Bluetooth 4.0, Bluetooth 5. The second interface optionally comprises a loudspeaker and/or a haptic feedback element for provision of an audio signal and/or haptic feedback to the user, respectively.

The wound dressing system may comprise a docking station forming an accessory device of the wound dressing system. The docking station may be configured to electrically and/or mechanically couple the monitor device to the docking station.

The docking station may comprise a docking monitor interface. The docking monitor interface may be configured for electrically and/or mechanically connecting the monitor device to the docking station. The docking monitor interface may be configured for wirelessly connecting the monitor device to the docking station. The docking monitor interface of the docking station may be configured to electrically and/or mechanically couple the docking station and the monitor device.

The docking monitor interface of the docking station may comprise, e.g. as part of a first connector of the docking monitor interface, a coupling part for forming a mechanical connection, such as a releasable coupling between the monitor device and the docking station. The coupling part may be configured to engage with a coupling part of the monitor device for releasably coupling the monitor device to the docking station.

The docking monitor interface of the docking station may comprise, e.g. as part of a first connector of the docking monitor interface, a plurality of terminals, such as two, three, four, five, six, seven or more terminals, for forming electrical connections with respective terminals of the monitor device. The docking monitor interface may comprise a ground terminal. The docking monitor interface may comprise a first terminal and/or a second terminal. The docking station may comprise a third terminal. The docking monitor interface may comprise a fourth terminal and/or a fifth terminal. The docking monitor interface optionally comprises a sixth terminal.

The present disclosure provides a method, performed in an accessory device, for communicating an operating state of a wound dressing comprising an absorbent core layer, which in turn enables a monitoring of the wound dressing/absorbent core layer at the accessory device. The accessory device comprises an interface configured to communicate with one or more devices of a wound dressing system. The one or more devices comprise a monitor device connected to a wound dressing, the wound dressing being placed on a skin surface of a user or on any additional seals. The method comprises obtaining (e.g. receiving and/or retrieving) monitor data from the monitor device. The monitor data is indicative of a condition of the wound dressing. The monitor data may be indicative of wetting of a distal surface of the absorbent core layer. The method comprises determining an operating state of the wound dressing based on the monitor data. The operating state is indicative of a degree of wetting of the absorbent core layer of the wound dressing and indicative of a wetting pattern of the absorbent core layer. The method comprises communicating the operating state of the wound dressing via the interface.

The condition of the wound dressing may refer to a level of a physical property of at least a part of the wound dressing, such as a level of wetting and/or temperature of at least one or more parts of the wound dressing, such as a level of a physical property of at least a layer of the wound dressing, such as a level of wetting and/or temperature of at least a layer of the wound dressing.

In one or more exemplary methods, the method may comprise determining an operating state of the wound dressing based on the monitor data. The operating state may be indicative of wetting of a distal surface of the absorbent core layer. An operating state in the present disclosure is indicative of the dynamic internal state of the wound dressing, related to the absorbent capacity or absorbent performance of the (absorbent core layer of the) wound dressing. Absorbent capacity or performance of of the wound dressing may be related to the condition inside the wound dressing (e.g. which may be affected by several factors such as wound condition (e.g. exudation rate, exudate composition, healing degree of wound) and/or malfunction of the wound dressing). One or more factors alone or in combination may impact the absorbent capacity or absorbent performance of the wound dressing. Absorbent capacity may be indicative of wear property, e.g. wear time and/or wear comfort. An operating state in the present disclosure is configured to indicate whether the wound dressing needs to be changed based on its adhesive performance (e.g. wear property, e.g. wear time and/or wear comfort). For example, the operating state may be indicative of the severity and/or imminence of the required change (e.g. low, medium, acute). The operating state may comprise N_OS operating states, where N_OS is an integer. The operating state may comprise a first operating state, a second operating state, a third operating state. Exemplary operating states may be indicative of good, check, change in X time, and/or change NOW.

The method comprises communicating (e.g. outputting, transmitting, and/or displaying) the operating state of the wound dressing via the interface, e.g. to the user and/or the one or more devices of the wound dressing system.

It is an advantage of the present disclosure that a user of a wound dressing, a caretaker or a health care professional is able to monitor and plan the use of the wound dressing. Communication of the operating states of the wound dressing is useful in helping to reduce the risk of a user experiencing leakage from a wound dressing (e.g. exudate material leakage from the wound dressing), which in turn helps in reducing risks of skin damage to a user and/or helps in providing improved healing conditions for the wound. In particular, determination and communication of operating state according to the present disclosure is performed based on monitor data indicative of a condition of the wound dressing which may not be visible to the user (because it is under the top layer of the wound dressing and/or inside the absorbent core layer). This results in providing a clear distinction or differentiation between different degrees of exudation for different section or zones of the wound.

The present disclosure provides an efficient, and easy-to-use monitoring of a wound dressing system with a high degree of comfort for a user while allowing derivation of an operating state based on monitor data that is not accessible or visible by the user or the health care professional. In other words, the disclosed method allows indication of the dynamic internal state of the wound dressing to a user, which supports the user in operating the wound dressing properly.

In one or more exemplary methods, the wound dressing comprises an electrode assembly comprising a plurality of sensor points configured to measure electrical properties of the absorbent core layer, such as electrical properties of a distal surface of the absorbent core layer, and wherein obtaining monitor data comprises obtaining data representative of the measurement of the electrical properties of the absorbent core layer, such as electrical properties of a distal surface of the absorbent core layer. The electrical properties may be indicative of a conductive path on the distal surface of the absorbent core layer, thereby indicative of wetting, and indicative of the condition of the wound dressing. In one or more exemplary methods, obtaining monitor data comprises obtaining data representative of the measurement of the electrical properties of the absorbent core layer. In other words, it may be envisaged that in one or more embodiments, the monitor data is indicative of the operating state of the wound dressing.

In one or more exemplary methods, the monitor data comprises wound data and/or parameter data. For example, the parameter data is derived based on wound data.

In one or more exemplary methods, the wound data and/or parameter data are indicative of resistance between electrodes, capacitance and/or inductance between electrodes and/or any change thereof. In one or more exemplary methods, the wound data and/or parameter data are indicative of a change in resistance, capacitance and/or inductance between electrodes. In one or more exemplary methods, the wound data and/or parameter data comprises timing information, such as timestamped data or information from which timing is derivable.

In one or more exemplary methods, the monitor data comprises localized monitor data with respect to a location and/or zone on the wound dressing, such as zone on the distal surface of absorbent core layer. The location and/or zone on the wound dressing may be related to a position on the absorbent core layer where electrical properties have been measured by the sensor points (electrode pairs). In one or more exemplary methods, obtaining the monitor data comprises obtaining (e.g. receiving from one or more devices in the wound dressing system, and/or retrieving from one or more devices in the wound dressing system) localized monitor data with respect to a location and/or zone on the wound dressing. In one or more exemplary methods, the localized monitor data may be with respect to a first location, a second location, a third location. In one or more exemplary methods, the localized monitor data may be with respect to a first zone, a second zone, and/or a third zone on the wound dressing. In one or more exemplary methods, the monitor data may comprise first localized monitor data indicative of condition at a first location of the absorbent core layer or at a first zone of the absorbent core layer, second localized monitor data indicative of condition at a second location of the absorbent core layer or at a second zone of the absorbent core layer.

In one or more exemplary methods, determining the operating state of the wound dressing based on the monitor data comprises determining a wetting pattern of the absorbent core layer, and determining a pattern type of the wetting pattern from a plurality of pattern types optionally based on the monitor data, such as based on the wound data and/or the parameter data (e.g. first parameter data and second parameter data), such as based on measurements obtained by the electrodes, e.g. measurements of resistance, capacitance and/or inductance, and /or timing information. The pattern type is optionally indicative of wetting pattern of the wound dressing and/or leakage risk of the wound dressing and/or indicative of the risk of skin damage to the user of the wound dressing system. In one or more exemplary methods, determining the operating state of the wound dressing based on the monitor data comprises determining one or more pattern types based on the first parameter data and second parameter data (and optionally a third parameter).

In one or more exemplary methods, determining a wetting pattern types may comprise selecting a wetting pattern type from a set of predefined wetting pattern types. The set of predefined wetting pattern types may comprise a number M of wetting pattern types, such as at least three wetting pattern types, at least four wetting pattern types, at least five wetting pattern types. The number M of wetting pattern types may be in the range from 4 to 100, such as from 5 to 20.

In one or more exemplary methods, determining the operating state of the wound dressing based on the monitor data comprises deriving the operating state based on the wetting pattern type, such as a first wetting pattern type, a second wetting pattern type, and/or a third wetting pattern type.

In one or more exemplary methods, determining one or more wetting pattern types comprises determining whether the parameter data including at least one of the first parameter data, the second parameter data, and optionally the third parameter data meets first criteria, and the wetting pattern type is set to the first wetting pattern type if the first criteria are met. For example, the first criteria may comprise a first primary criterion based on the first parameter data, a first secondary criterion based on the second parameter data, and optionally a first tertiary criterion based on the third parameter data. For example, the first criteria may be given by e.g.:
(p_1_1 < TH_1_1) and (p_i_1 > TH_1_2) for all i =2, ..., N,
wherein p_1_1 is a first primary parameter of the first parameter data (resistance between two electrodes of first sensor point, TH_1_1 is a first primary threshold value, p_i_1 is an i'th primary parameter of the i'th parameter data (resistance between two electrodes of the i'th sensor point and TH_1_2 is a first secondary threshold value and wherein the first wetting pattern type may be indicative of very low wetting of the absorbent core layer of the wound dressing and/or wetting of a first zone, section or region of the (distal surface of) absorbent core layer.

In one or more exemplary methods, determining one or more wetting pattern types comprises determining whether at least one of the first parameter data, the second parameter data, and the third parameter data meets second criteria, and the wetting pattern type is set to the second type if the second criteria are met. For example, the second criteria may be given by e.g.:
(p_2_1 < TH_1_1) and (p_i_1 > TH_1_2) for all i = 1, 3, 4, ..., N,
wherein p_i_1 is an i'th primary parameter of the i'th parameter data (resistance between two electrodes of the i'th sensor point, TH_1_1 and TH_1_2 are resistance thresholds, and wherein the second wetting pattern type is indicative very low wetting of the absorbent core layer of the wound dressing and/or wetting of a second zone, section or region of the (distal surface of) absorbent core layer.

In one or more exemplary methods, determining one or more wetting pattern types comprises determining whether at least one of the first parameter data, the second parameter data, and the third parameter data meets third criteria. The wetting pattern type may be set to the third wetting pattern type if the third criteria are met. In one or more exemplary methods, the third criteria are given by
(p_3_1 < TH_1_1) and (p_i_1 > TH_1_2) for all i = 1, 2, 4, ..., N,
wherein p_i_1 is an i'th primary parameter of the i'th parameter data (resistance between two electrodes of the i'th sensor point, TH_1_1 and TH_1_2 are resistance thresholds, and wherein the second wetting pattern type is indicative very low wetting of the absorbent core layer of the wound dressing and/or wetting of a third zone, section or region of the (distal surface of) absorbent core layer.

In one or more exemplary methods, determining one or more wetting pattern types comprises determining a wetting pattern type if a change criterion is fulfilled. The change criterion may be based on the parameter data optionally including the first parameter data, the second parameter data and/or the third parameter data. The change criterion may be fulfilled if parameter data changes, e.g. if a change in parameter data is larger than a change threshold. Thus, wetting pattern type determination may be conditional on a change in the parameter data, in turn leading to an optimum use of power or battery resources in the monitor device and/or accessory device.

In one or more exemplary methods, determining one or more wetting pattern types based on the monitor data comprises comparing different parameter data, such as first parameter data and second parameter data. In one or more exemplary methods, determining one or more wetting pattern types based on the monitor data comprises identifying a wetting pattern type based on the comparison of parameter data. In one or more exemplary methods, determining one or more wetting pattern types based on the monitor data comprises comparing first parameter data, second parameter data, and third parameter data. In one or more exemplary methods, determining one or more wetting pattern types based on the monitor data comprises identifying a wetting pattern type based on the comparison. For example, an exemplary criterion, such as one or more of a first criterion (of the first criteria), a second criterion (of the second criteria) and a third criterion (of the third criteria), may be based on a difference and/or rate between parameter data and/or parameters derived from parameter data. For example, determining a wetting pattern comprises determining a first tertiary parameter, also denoted p_1_3, indicative of a time delay between changes in the first parameter data and the second parameter data, such as a time delay between the first parameter data reaching (or being below) a first threshold and the second parameter data reaching (or being below) a second threshold. A high time delay (p_1_3 > TH_D_1) may be indicative of low exudation rate. One or more criteria may be based on the first tertiary parameter. For example, determining a wetting pattern comprises determining a third tertiary parameter, also denoted p_3_3, indicative of a time delay between changes in the third parameter data and the fourth parameter data, such as a time delay between the third parameter data reaching (or being below) a third threshold and the fourth parameter data reaching (or being below) a fourth threshold. A high time delay (p_3_3 > TH_D_3) may be indicative of low exudation rate, while a low time delay (p_3_3 < TH_D_1) may be indicative of high exudation rate. One or more criteria may be based on the third tertiary parameter.

In one or more exemplary methods, the interface comprises an audio interface, a visual interface, and/or a transceiver module.

In one or more exemplary methods, communicating the operating state of the wound dressing comprises displaying, on a visual interface (e.g. a display) of the accessory device, a user interface comprising a user interface object representative of the operating state, such as a first user interface object representative of a first operating state, a second user interface object representative of a second operating state, and/or a third user interface object representative of a third operating state. The user interface object may be representative of one or more wetting pattern types determined, such as a first wetting pattern type, a second wetting pattern type, and/or a third wetting pattern type. The user interface object may be representative of a degree of wetting of the absorbent core layer, e.g. a relative measure of how many sensor points of the N sensor points have experienced a drop in resistance, e.g. to a resistance less than a threshold, between two electrodes of the respective sensor point.

The user interface may comprise a number of user interface elements, e.g. arranged in a matrix configuration corresponding to a distribution of sensor points on the distal surface or side of the absorbent core layer.

In one or more exemplary methods, communicating the operating state of the wound dressing comprises determining a representation of the operating state of the wound dressing and outputting the representation on a user interface of the accessory device.

The user interface may comprise a user interface element indicative of a degree of wetting of the wound dressing/absorbent core layer or distal surface or side of the absorbent core layer.

In one or more exemplary methods, the user interface object representative of the operating state comprises one or more of a first user interface object, and a second user interface object, wherein the first and/or second user interface objects are indicative of one or more of a first operating state, a second operating state and a third operating state. For example, the user interface object may comprise one or more visual indicators representative of the operating state, such as a first visual indicator, a second visual indicator, a third visual indicator. For example, the visual indicator may be in a shape of two or more concentric rings indicating one or more of: the wetting pattern type, severity, imminence, and/or position of the wetting pattern type in the wound dressing. For example, two or more concentric rings may be arranged to surround a central circle representative of a center region of the absorbent core layer. For example, the two or more concentric rings around the central circle may visually change to reflect the wetting pattern type, severity, imminence, and/or position of the wetting pattern type in the wound dressing. The visual change may be performed by a change of one or more of: colour, shape, blurring, and animation. For example, the two or more concentric rings may be arranged so as to be split into a plurality of angular visual indicators, wherein an angular visual indicator is indicative of the angular position of the wetting pattern type on the wound dressing or absorbent core layer. For example, the visual indicators may be a text prompt indicating to the user the dynamic internal operating state of the wound dressing.

In one or more exemplary methods, communicating the operating state of the wound dressing comprises notifying the user via the interface, such as by displaying a notification on a display of the accessory device. The notification may comprise the user interface object representative of the operating state of the wound dressing. The notification may comprise a notification indicator to open an application related to the wound dressing. The method may comprise detecting an input on the notification indicator, in response to the input, opening the application related to the wound dressing, and in response to the opening of the application, displaying the operating state.

In one or more exemplary methods, the wound dressing system comprises a server device. In one or more exemplary methods, communicating the operating state of the wound dressing comprises communicating the operating state of the wound dressing to the server device. For example, communicating the operating state of the wound dressing to the server device may comprise transmitting a message comprising the operating state to the server device from the accessory device.

The present disclosure provides an accessory device, the accessory device forming part of a wound dressing system. The accessory device comprises a memory; a processor coupled to the memory; and an interface, coupled to the processor. The interface is configured to communicate with one or more devices of the wound dressing system. The one or more devices comprises a monitor device connectable to a wound dressing configured to be placed on a skin surface of a user or on any additional seals. The interface is configured to obtain monitor data from the monitor device, such as to receive or retrieve the monitor data from the one or more devices. The monitor data is indicative of a condition of the wound dressing, such as a condition of a distal side of the absorbent core layer of the wound dressing.

The processor is configured to determine an operating state of the wound dressing based on the monitor data. The operating state is indicative of a degree of wetting of the absorbent core layer of the wound dressing and indicative of a wetting pattern of the absorbent core layer. The processor may be configured to determine the operating state by determining a wetting pattern type. The wetting pattern type may be based on one or more, such as all, of first monitor data, second monitor data, and third monitor data, such as first parameter data, second parameter data and the third parameter data. The wetting pattern type may be based on the fourth parameter data.

In one or more exemplary accessory devices, determination of a wetting pattern type may comprise selecting a wetting pattern type from a set of predefined wetting pattern types. The set of predefined wetting pattern types may comprise a number M of wetting pattern types, such as at least three wetting pattern types, at least four wetting pattern types, at least five wetting pattern types. The number M of wetting pattern types may be in the range from 4 to 100, such as from 5 to 20. The number M of wetting pattern types may be less than the number N of sensor points.

The memory may be configured to store the monitor data and/or the operating state.

The interface is configured to communicate the operating state of the wound dressing to the user via the interface, such as an audio interface, a visual interface, and/or a transceiver module.

In one or more accessory devices, the interface is configured to communicate the operating state of the wound dressing by displaying, on a visual interface (e.g. a display) of the accessory device, a user interface comprising a user interface object representative of the operating state, such as a first user interface object representative of a first operating state, a second user interface object representative of a second operating state, and/or a third user interface object representative of a third operating state. The user interface object may be representative of one or more wetting pattern types determined, such as a first wetting pattern type, a second wetting pattern type, and/or a third wetting pattern type. In one or more accessory devices, the user interface object representative of the operating state comprises one or more of a first user interface object, and a second user interface object, wherein the first and/or second user interface objects are indicative of one or more of a first operating state, a second operating state and a third operating state. For example, the user interface object may comprise one or more visual indicators representative of the operating state, such as a first visual indicator, a second visual indicator, a third visual indicator.

The present disclosure provides a wound dressing system comprising a wound dressing, an accessory device and a monitor device, the wound dressing comprising an absorbent core layer. The accessory device is configured to perform any of the methods disclosed herein.

The present disclosure provides a computer readable storage medium storing one or more programs, the one or more programs comprising instructions, which when executed by an accessory device with an interface, a memory and a processor cause the accessory device to perform any of the methods disclosed herein.

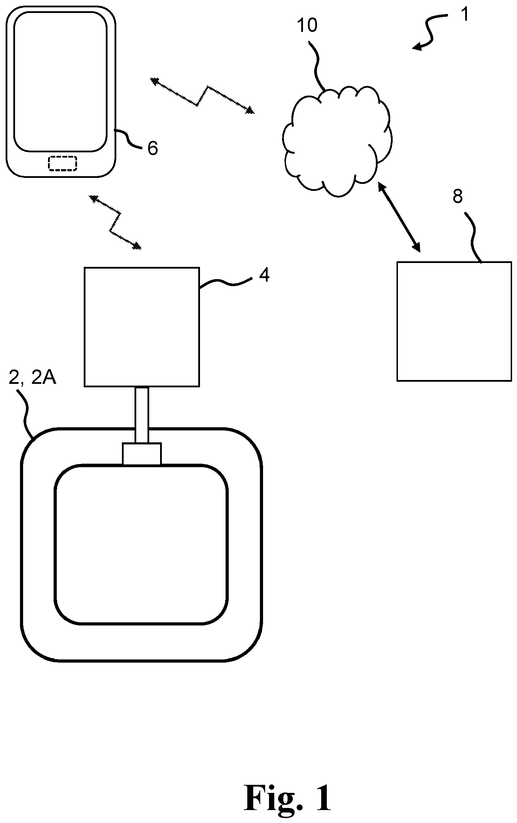

Fig. 1 illustrates an exemplary wound dressing system. The wound dressing system 1 comprises a wound dressing 2, 2A, a monitor device 4, and optionally an accessory device 6 (mobile telephone). The monitor device 4 is connectable to the wound dressing via respective connectors of the monitor device 4 and wound dressing 2, 2A. The monitor device 4 is configured for wireless communication with the accessory device 6. Optionally, the accessory device 6 is configured to communicate with a server device 8 of the wound dressing system 1, e.g. via network 10. The server device 10 may be operated and/or controlled by the wound dressing manufacturer and/or a service centre. Wound data and/or parameter data based on the wound data are obtained from electrodes/sensors of the wound dressing 2, 2A with the monitor device 4. The monitor device 4 processes the wound data and/or parameter data based on the wound data to determine monitor data that are transmitted to the accessory device 6. In the illustrated wound dressing system 1, the accessory device 6 is a mobile phone, however the accessory device 6 may be embodied as another handheld device, such as a tablet device, or a wearable, such as a watch or other wrist-worn electronic device. Accordingly, the monitor device 4 is configured to determine and transmit monitor data to the accessory device 6.

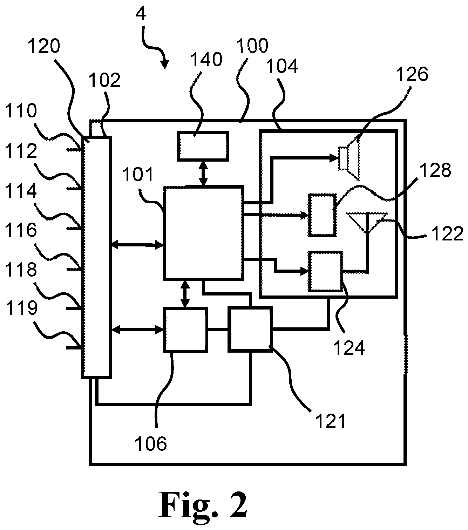

Fig. 2 is a schematic block diagram of an exemplary monitor device. The monitor device 4 comprises a monitor device housing 100, a processor 101, and one or more interfaces, the one or more interfaces including a first interface 102 (wound dressing interface) and a second interface 104 (accessory interface). The monitor device 4 comprises a memory 106 for storing wound data and/or parameter data based on the wound data. The memory 106 is connected to the processor 101 and/or the first interface 102. The first interface 102 is configured as a wound dressing interface for electrically and/or mechanically connecting the monitor device 4 to the wound dressing, e.g. wound dressing 2. The first interface 102 comprises a plurality of terminals for forming electrical connections with respective terminals of the wound dressing. The first interface 102 comprises a first terminal 110, a second terminal 112 and a third terminal 114. The first interface 102 optionally comprises a fourth terminal 116, a fifth terminal 118 and/or sixth terminal 119. The first interface 102 of the monitor device 4 comprises a coupling part 120 for forming a mechanical connection, such as a releasable coupling, between the monitor device 4 and the wound dressing. The coupling part 120 and terminals 110, 112, 114, 116, 118, 119 of the first interface 102 form (at least part of) a first connector of the monitor device 4. Terminals 110, 112, and 114 may be respectively coupled to first electrodes 210A, 210B, 210C via the monitor interface of the wound dressing, and terminals 116, 118, 119 may be respectively coupled to second electrodes 212A, 212B, 212C via the monitor interface of the wound dressing.

The monitor device 4 comprises a power unit 121 for powering the monitor device and active components thereof, i.e. the power unit 121 is connected to the processor 101, the first interface 102, the second interface 104, and memory 106. The power unit comprises a battery and optionally charging circuitry. The charging circuitry is optionally connected to the battery and terminals of the first interface 102 for charging the battery via terminals of the first interface, e.g. terminals of the first connector.

The second interface 104 of monitor device 4 is configured as an accessory interface for connecting the monitor device 4 to one or more accessory devices such as accessory device 6. The second interface 104 comprises an antenna 122 and a wireless transceiver 124 configured for wireless communication with accessory device(s). Optionally, the second interface 104 comprises a loudspeaker 126 and/or a haptic feedback element 128 for provision of respective audio signal and/or haptic feedback to the user.

The monitor device 6 optionally comprises a sensor unit 140 connected to the processor 101. The sensor unit 140 comprises a temperature sensor for feeding temperature data to the processor and/or a G-sensor or accelerometer for feeding acceleration data to the processor 101.

The processor 101 is configured to apply a processing scheme, and the first interface 102 is configured for collecting wound data from the wound dressing coupled to the first interface, the wound data comprising wound data from sensor points (electrode pairs) of the wound dressing. The wound data may comprise first wound data WD_1 from a first electrode pair (first sensor point) of the wound dressing, second wound data WD_2 from a second electrode pair (second sensor point) of the wound dressing, and third wound data WD_3 from a third electrode pair (third sensor point) of the wound dressing. In the illustrated monitor device, the processor is configured to collect, WD_1, WD_2, WD_3, ..., WD_9 from nine sensor points of the wound dressing formed by nine electrode pairs being combinations of a first electrode of the first set of first electrodes and a second electrode of the second set of second electrodes. The wound data may be stored in the memory 106 and/or processed in the processor 101 in order to obtain parameter data based on the wound data. The parameter data may be stored in the memory 106. The processor 101 is configured to apply a processing scheme, wherein to apply a processing scheme comprises obtain first parameter data P_1 including p_1_1 being the resistance between the two electrodes forming the first sensor point based on the first wound data; obtain second parameter data P_2 including p_2_1 being the resistance between the two electrodes forming the second sensor point based on the second wound data; obtain third parameter data P_3 including p_3_1 being the resistance between the two electrodes forming the third sensor point based on the third wound data. In other words, the processor 101 is configured to obtain parameters p_1_1, p_2_1, ..., p_9_1 being resistances based on respective wound data WD_1, WD_2, ..., WD_9 obtained between the two electrodes forming the respective sensor points. To apply a processing scheme comprises to determine an operating state of the wound dressing based on one or more, e.g. all, of the parameter data P_1, P_2, ..., P_9 including p_1_1, p_2_1, ..., p_9_1. The operating state is optionally indicative of a degree of wetting or wetting pattern on the distal side of the absorbent core layer of the wound dressing.

The monitor device 4 is optionally configured to, in accordance with a determination that the operating state is a first operating state of the wound dressing, transmit a first monitor signal comprising monitor data indicative of the first operating state of the wound dressing via the second interface, the monitor data optionally including including a pattern representation of the wetting pattern and/or the degree of wetting. The pattern representation may comprise a pattern type identifier and/or pattern data indicative of or comprising parameter data. Optionally, the monitor device 4 may be configured to, in accordance with a determination that the operating state is a second operating state of the wound dressing, transmit a second monitor signal comprising monitor data indicative of the second operating state of the wound dressing via the second interface, the monitor data optionally including including a pattern representation of the wetting pattern and/or the degree of wetting. The pattern representation may comprise a pattern type identifier and/or pattern data indicative of or comprising parameter data.

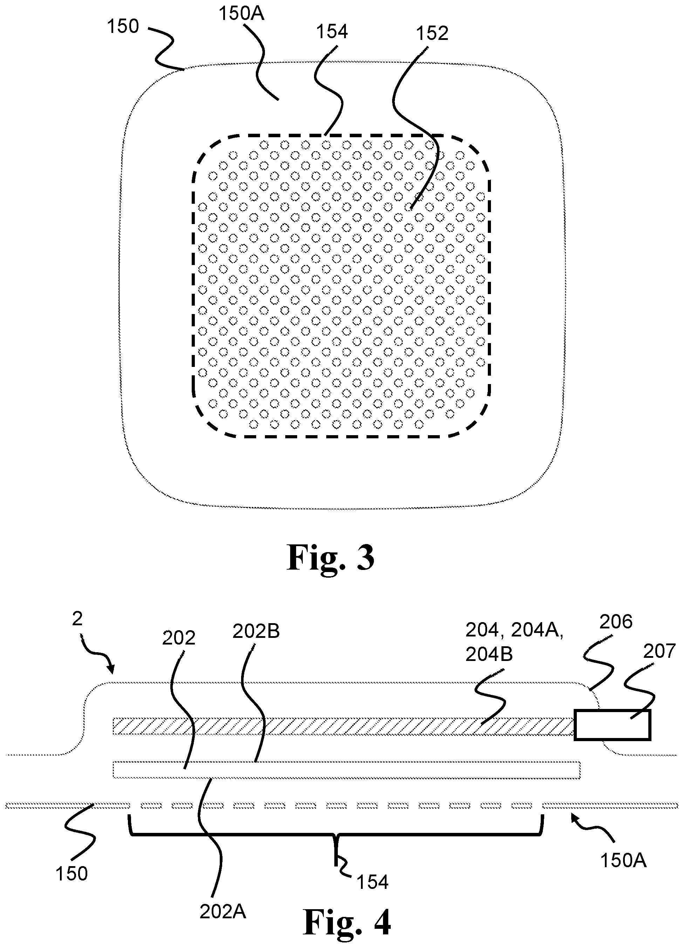

Fig. 3 shows a proximal view of an exemplary first adhesive layer of the wound dressing. The first adhesive layer 150 has a proximal surface 150A configured for attachment of the wound dressing to the skin surface of a user. The first adhesive layer has perforations or through-going openings 152 arranged within absorbing region 154 for allowing exudate from the wound to flow through the perforations of first adhesive layer 150 to be absorbed by absorbent core layer arranged on the distal side of the first adhesive layer.

Fig. 4 is a schematic cross-section of an exemplary wound dressing. The wound dressing 2 comprises a first adhesive layer 150 with a proximal surface 150A configured for attachment of the wound dressing to the skin surface of a user. The wound dressing 2 may form part of wound dressing system 1. The wound dressing 2 comprises an absorbent core layer 202 with a proximal surface 202A and a distal surface 202B; an electrode assembly 204, 204A, 204B comprising a plurality of electrodes arranged on a distal side of the absorbent core layer 202; and a top layer 206 at least partly on a distal side of the electrode assembly. The wound dressing comprises a monitor interface 207 configured to connect electrodes of the wound dressing to terminals of monitor device.

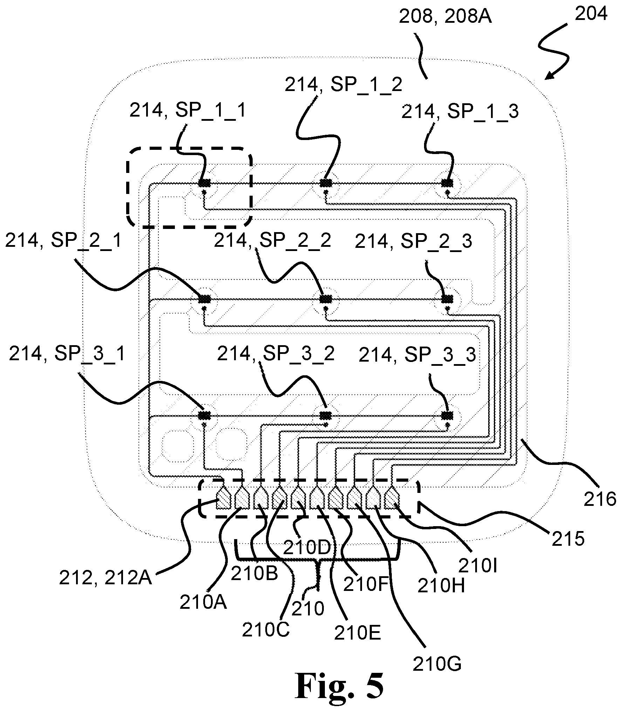

Fig. 5 is a proximal view of an exemplary and schematic electrode assembly of wound dressing 2. The electrode assembly 204 comprises a first support layer 208 and a plurality of electrodes printed on a proximal surface 208A of the first support layer, i.e. the plurality of electrodes is arranged on a distal side of the absorbent core layer of the wound dressing. 202. The plurality of electrodes comprises a first set 210 of nine first electrodes 210A, ..., 2101 and a second set 212 of a second electrode 212A. A sensing part of a first electrode and a sensing part of a second electrode forms a sensor point. The electrode assembly 204 comprises nine sensor points 214 arranged on the proximal surface of the first support layer and distributed along a distal surface of the absorbent core layer, e.g. as shown with nine sensor points arranged in a 3x3 matrix sensor point configuration.

The electrode assembly 204 comprises a first masking layer 216 covering and isolating electrode parts of the first electrodes 210A, , 2101 and the second electrode 212A. The first masking layer 216 is printed on the first support layer/electrodes and comprises a number of sensor point openings to form respective sensor points of the electrode assembly by exposing sensing parts of first electrodes 21 0A, ..., 2101 and second electrode 212A.

The second electrode 212A operates as a reference electrode (ground) for the first electrodes 210A, ..., 2101 and forms a part of the respective sensor points 214 of electrode assembly 204. The sensor points 214 are arranged with a distance between two neighbouring sensor points in the range from 3 mm to 50 mm, e.g. with a center-to-center distance of 30 mm.

Each electrode 210A, ..., 2101 , 212 has a respective connection part (connection parts indicated with dashed box 215 for forming a connection to monitor device via a wired or wireless monitor interface of the wound dressing.

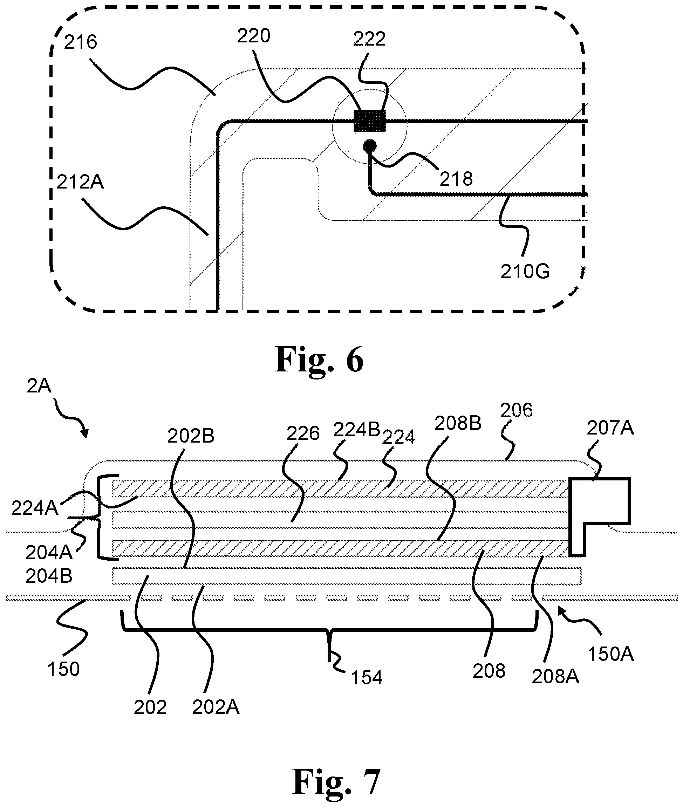

Fig. 6 shows a more detailed view of a sensor point 214 of an electrode assembly, e.g. electrode assembly 204, see dashed box in Fig. 5. The sensor point 214 is formed by a first sensing part 218 of a first electrode and a second sensing part 220 of a second electrode. The first sensing part 218 and the second sensing part 220 are exposed to the absorbent core layer of the wound dressing through sensor point opening 222 of the first masking layer 216. Thus, exudate or other fluid reaching the distal surface of the absorbent core layer short-circuits the first sensing part 218 and the second sensing part 220. In the illustrated electrode assembly, the sensor point opening 222 is circular with a radius in the range from 2 to 10 mm.

Fig. 7 is a schematic cross-section of an exemplary wound dressing. The wound dressing 2A comprises a first adhesive layer 150 with a proximal surface 150A configured for attachment of the wound dressing to the skin surface of a user. The wound dressing 2A may form part of wound dressing system 1. The wound dressing 2A comprises an absorbent core layer 202 with a proximal surface 202A and a distal surface 202B; and an electrode assembly 204A comprising a plurality of electrodes arranged on a distal side of the absorbent core layer 202, wherein the plurality of electrodes comprises a first set of first electrodes arranged on a proximal surface 208A and/or distal surface 208B of first support layer 208. The electrode assembly 204A comprises a second support layer 224, and the plurality of electrodes comprises a second set of second electrodes arranged on a proximal surface and/or distal surface of second support layer 208. The electrode assembly 204A comprises a spacing layer 226 arranged between the first support layer 208 and the second support layer 224. Further, a top layer 206 is arranged at least partly on a distal side of the electrode assembly 204A. The wound dressing 2A comprises a monitor interface 207A configured to connect electrodes of the wound dressing to terminals of monitor device.

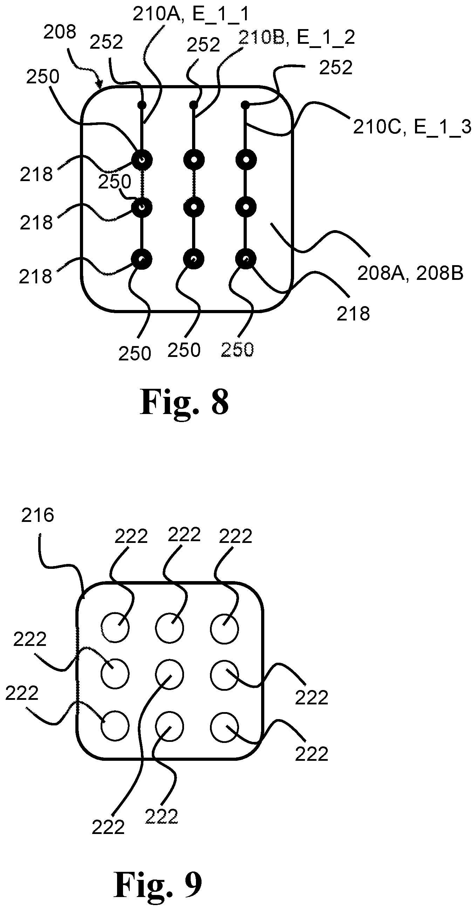

Fig. 8 shows a proximal view of an exemplary first support layer 208, e.g. of electrode assembly 204, electrode assembly 204A, electrode assembly 204B. The electrode assembly comprises three first electrodes 210A, 210B, 210C printed on the proximal surface 208A of the first support layer 208, wherein each electrode 210A, 210B, 210C comprises three first sensing parts 218 exposed through respective sensor point openings of first masking layer, see Fig. 9. The first support layer 208 has a plurality of sensor point openings 250 for allowing exudate to pass through the first support layer (from proximal side to distal side) and reach sensing parts of second electrodes arranged on the distal side or distal surface of the first support layer 208. Each sensor point opening 250 is optionally centered in a respective first sensing part 218 of a first electrode. Each first electrode 210A, 210B, and 210C has a connection part 252 for connection to or forming part of a monitor interface of the wound dressing. In one or more exemplary electrode assemblies, e.g. electrode assemblies 204A, 204B shown in Fig. 7, the first electrodes 210A, 21 0B, 210C may be printed on the distal surface 208B of the first support layer 208.

Fig. 9 shows a proximal view of an exemplary first masking layer 216 of electrode assembly with first support layer of Fig. 8. The first masking layer 216 is printed on the proximal surface/distal surface of first support layer partly covering first electrodes 210A, 210B, and 210C of the electrode assembly. The first masking layer comprises nine sensor point openings 222 arranged to fit a 3x3 matrix sensor point configuration and respectively aligned with first sensing parts 218 of first electrodes 210A, 210B, 210C.

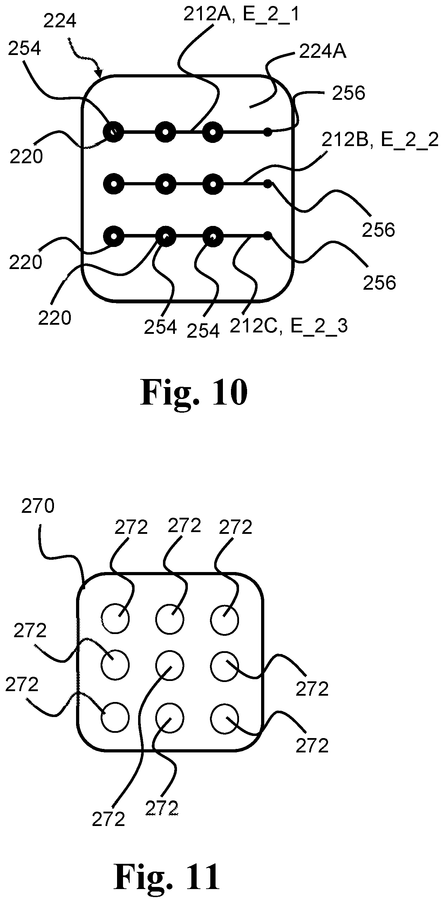

Fig. 10 shows a proximal view of an exemplary second support layer 224, e.g. of electrode assembly 204A. The electrode assembly comprises three second electrodes 212A, 212B, 212C printed on proximal surface 224A of the second support layer 224, wherein each electrode 212A, 212B, 212C comprises three second sensing parts 220 exposed through respective sensor point openings of second masking layer, see Fig. 11. Optionally, the second support layer 224 has a plurality of sensor point openings 254 for allowing exudate to pass through the second support layer (from proximal side to distal side). Each sensor point opening 254 is optionally centered in a respective second sensing part 220 of a second electrode. Each second electrode 212A, 212B, and 212C has a connection part 256 for connection to or forming part of a monitor interface of the wound dressing.

Fig. 11 shows a proximal view of an exemplary second masking layer 270 of electrode assembly 204A. The second masking layer 270 is printed on the proximal surface 224A of the second support layer 224 partly covering second electrodes 212A, 212B, and 212C of the electrode assembly. The second masking layer 270 comprises nine sensor point openings 272 arranged to fit a 3x3 matrix sensor point configuration and respectively aligned with second sensing parts 220 of second electrodes 212A, 212B, 212C. Referring back to Figs. 7-11, the first sensing parts 218 of first electrodes 210A, 210B, 210C are respectively aligned with a second sensing part 220 of second electrodes 212A, 212B, 212C to form sensor points.

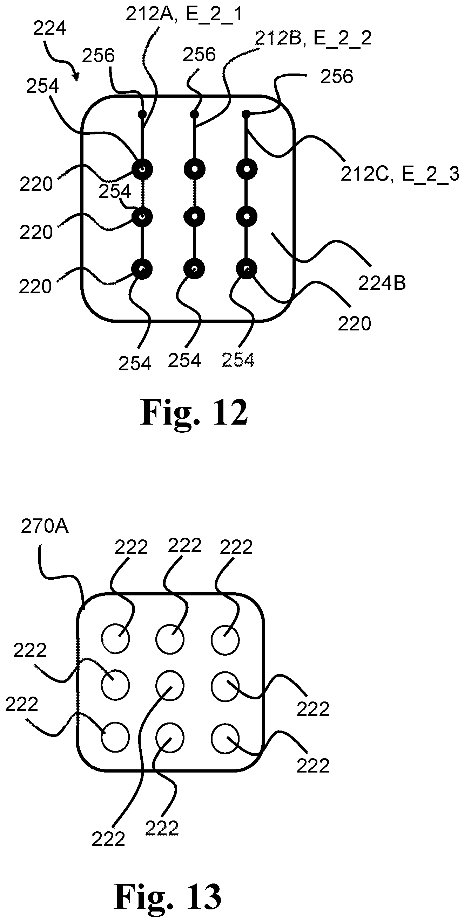

Fig. 12 shows a distal view of an exemplary second support layer 224, e.g. of electrode assembly 204B. The electrode assembly comprises three second electrodes 212A, 212B, 212C printed on distal surface 224B of the second support layer 224, wherein each electrode 212A, 212B, 212C comprises three second sensing parts 220 optionally exposed through respective sensor point openings of second masking layer, see Fig. 13. The second support layer 224 has a plurality of sensor point openings 254 for allowing exudate to pass through the second support layer (from proximal side to distal side) and reach second sensing parts 220 of second electrodes arranged on the distal side or distal surface of the second support layer 224. Each sensor point opening 254 is optionally centered in a respective second sensing part 220 of a second electrode. Each second electrode 212A, 212B, and 212C has a connection part 256 for connection to or forming part of a monitor interface of the wound dressing.

Fig. 13 shows a proximal view of an exemplary and optional second masking layer 270A of electrode assembly 204B or a second masking layer 270A of electrode assembly 204. The second masking layer 270A is printed on the distal surface 224B of the second support layer 224 or on distal surface 208B of the first support layer 208 partly covering second electrodes 212A, 212B, and 212C of the electrode assembly. The second masking layer 270A comprises nine sensor point openings 272 arranged to fit a 3x3 matrix sensor point configuration and respectively aligned with second sensing parts 220 of second electrodes 212A, 212B, 212C. Referring back to Figs. 4 and 7-11, the first sensing parts 218 of first electrodes 210A, 210B, 210C are respectively aligned with a second sensing part 220 of second electrodes 212A, 212B, 212C to form sensor points.

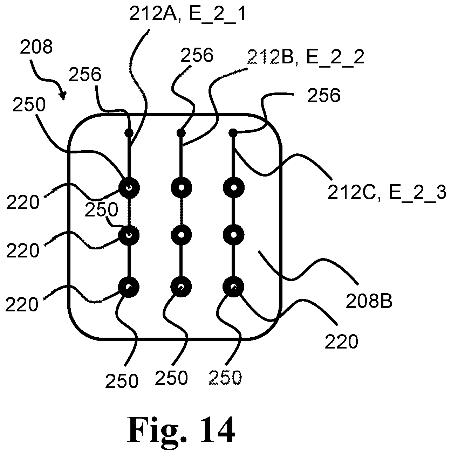

Fig. 14 shows a distal view of an exemplary first support layer 208 of an electrode assembly, e.g. of electrode assembly 204. The electrode assembly comprises three second electrodes 212A, 212B, 212C printed on distal surface 224B of the first support layer 208, wherein each second electrode 212A, 212B, 212C comprises three second sensing parts 220 optionally exposed through respective sensor point openings of second masking layer, see Fig. 13. The first support layer 208 has a plurality of sensor point openings 250 for allowing exudate to pass through the first support layer (from proximal side to distal side) and reach second sensing parts 220 of second electrodes arranged on the distal side or distal surface of the first support layer 208. Each sensor point opening 250 is optionally centered in a respective second sensing part 220 of a second electrode. Each second electrode 212A, 212B, and 212C has a connection part 256 for connection to or forming part of a monitor interface of the wound dressing.

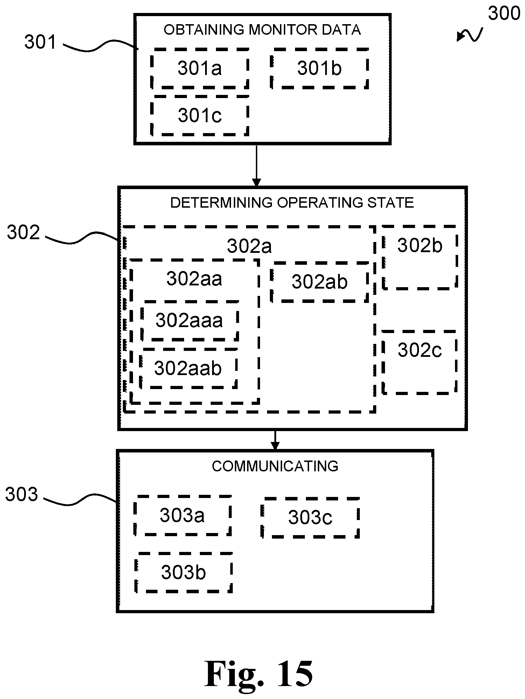

Fig. 15 illustrates an exemplary method 300 of communicating the operating state according to the present disclosure. The method 300 is performed in an accessory device, for communicating the operating state of the wound dressing, which in turn enables a monitoring of the wound dressing at the accessory device. The accessory device comprises an interface configured to communicate with one or more devices of a wound dressing system. The one or more devices comprise a monitor device, and/or a wound dressing configured to be placed on a skin surface of a user or on any additional seals. The method 300 comprises obtaining 301 (e.g. receiving and/or retrieving) monitor data from the one or more devices. The monitor data is indicative of a condition of the wound dressing. The condition of the wound dressing may refer to a level of a physical property of at least a part of the wound dressing, such as a level or presence of wetting and/or temperature of at least a part of the wound dressing, such as a level of a physical property of at least a layer of the wound dressing, such as a level or presence of wetting and/or temperature of at least a layer of the wound dressing.

In one or more exemplary methods, the method 300 may comprise determining 302 an operating state of the wound dressing based on the monitor data. The operating state may be indicative of a degree of wetting of the absorbent core layer of the wound dressing. An operating state in the present disclosure is indicative of the dynamic internal state of the wound dressing, related to the absorbent capacity and/or absorbent performance of the wound dressing. Absorbent performance of the wound dressing may be related to the condition inside or on surfaces of the absorbent core layer of the wound dressing. One or more factors alone or in combination impact the absorbent performance of the wound dressing. Absorbent performance may be indicative of wear property, e.g. wear time and/or wear comfort. An operating state in the present disclosure is optionally configured to indicate whether the wound dressing needs to be changed based on its absorbent performance (e.g. wear property, e.g. wear time and/or wear comfort). For example, the operating state may be indicative of the severity and/or imminence of the required change (e.g. low, medium, acute). The operating state may comprise N_OS operating states, where N_OS is an integer. The operating state may comprise a first operating state, a second operating state, a third operating state (e.g. good, check, change in X time, change NOW.

The method comprises communicating 303 the operating state of the wound dressing via the interface to the user or to the one or more devices. Communicating 303 may comprise outputting, transmitting and/or displaying.

In one or more exemplary methods, obtaining 301 the monitor data comprises obtaining 301a parameter data indicative of resistances measured on a distal surface of the absorbent core layer of the wound dressing. The resistances may be seen as representative of conductive paths in the absorbent core layer, such as across the distal surface of the absorbent core layer. In other words, the condition may be seen as a condition of the absorbent core layer.

In one or more exemplary methods, the wound dressing comprises one or more electrodes configured to measure electrical properties of the absorbent core layer. The electrical properties may be indicative of a conductive path in or on a surface of the absorbent core layer, thereby indicative of the condition of the wound dressing. In one or more exemplary methods, obtaining 301 monitor data comprises obtaining 301b data representative of the measurement of the electrical properties of the absorbent core layer of the wound dressing.

In one or more exemplary methods, the monitor data comprises wound data and/or parameter data. For example, the parameter data is derived based on wound data. In one or more exemplary methods, obtaining 301 monitor data comprises obtaining 301c wound data and/or parameter data. In one or more exemplary methods, obtaining 301 monitor data comprises obtaining the wound data and /or parameter data indicative of resistance between electrodes, capacitance and/or inductance between electrodes and/or any change thereof. In one or more exemplary methods, the wound data and/or parameter data are indicative of a change in resistance, capacitance and/or inductance between electrodes. In one or more exemplary methods, obtaining 301 monitor data comprises obtaining the wound data and/or parameter data comprising timing information, such as timestamped data or information from which timing is derivable.

In one or more exemplary methods, the monitor data comprises localized monitor data with respect to a location and/or zone, such as a sensor point, on the wound dressing. The location and/or zone on the wound dressing may be related to a position on the absorbent core layer where electrical properties have been measured by the one or more electrodes. In one or more exemplary methods, obtaining 301 the monitor data comprises obtaining (e.g. receiving from one or more devices in the wound dressing system, and/or retrieving from one or more devices in the wound dressing system) localized monitor data with respect to a location and/or zone on the wound dressing/absorbent core layer. In one or more exemplary methods, the localized monitor data may be with respect to a first location, a second location, a third location. In one or more exemplary methods, the localized monitor data may be with respect to a first zone, a second zone, and/or a third zone on the wound dressing/absorbent core layer. In one or more exemplary methods, the monitor data may comprise first localized monitor data indicative of condition at a first location of the first adhesive layer of the wound dressing or at a first zone of the absorbent core layer, second localized monitor data indicative of condition at a second location of the absorbent core layer or at a second zone of the absorbent core layer.

In one or more exemplary methods, determining 302 the operating state of the wound dressing based on the monitor data comprises determining 302a a wetting pattern type based on the monitor data, such as based on the wound data and/or the parameter data (e.g. first parameter data and second parameter data), such as based on measurements obtained by the electrodes, such as measurements of resistance, capacitance and/or inductance, such as timing information, of the electrode assemby. The wetting pattern type is optionally indicative of absorbent failure or saturation of the wound dressing and/or leakage risk of the wound dressing and/or indicative of the risk of skin damage to the user of the wound dressing system. The wetting pattern type is optionally indicative of healing conditions of the wound. In one or more exemplary methods, determining 302 the operating state of the wound dressing based on the monitor data comprises determining a wetting pattern type based on the first parameter data and second parameter data (and optionally a third parameter).

In one or more exemplary methods, determining 302a a wetting pattern type may comprise selecting a wetting pattern type from a set of predefined wetting pattern types. The set of predefined wetting pattern types may comprise a number M of wetting pattern types, such as at least three wetting pattern types, at least four wetting pattern types, at least five wetting pattern types. The number M of wetting pattern types may be in the range from 4 to 100, such as from 5 to 20.

In one or more exemplary methods, comparing 302aa the first parameter data and/or the second parameter data comprises determining 302aaa whether the first parameter data and/or the second parameter data satisfy one or more criteria, and identifying 302aab the wetting pattern type based on the determination.

In one or more exemplary methods, determining 302 the operating state of the wound dressing based on the monitor data comprises determining 302b a degree of wetting of the absorbent core layer based on the monitor data.

In one or more exemplary methods, determining 302 the operating state of the wound dressing based on the monitor data comprises deriving 302c the operating state based on the one or more (pre-defined) wetting pattern types, such as a first wetting pattern type, a second wetting pattern type, and/or a third wetting pattern type.

In one or more exemplary methods, communicating 303 the operating state of the wound dressing comprises displaying 303a, on a visual interface (e.g. a display) of the accessory device, a user interface comprising a user interface object representative of the operating state, such as a first user interface object representative of a first operating state, a second user interface object representative of a second operating state, and/or a third user interface object representative of a third operating state. The user interface object may be representative of a wetting pattern and/or a wetting pattern type determined. The user interface object may be representative of a degree of wetting of the absorbent core layer. For example, the user interface object may comprise one or more visual indicators representative of the operating state, such as a first visual indicator, a second visual indicator, a third visual indicator.

In one or more exemplary methods, communicating 303 the operating state of the wound dressing comprises notifying 303b the user via the interface, such as by displaying a notification on a display of the accessory device. The notification may comprise the user interface object representative of the operating state. The notification may comprise a notification indicator to open an application related to the wound dressing. The method may comprise detecting an input on the notification indicator, in response to the input, opening the application related to the wound dressing, and in response to the opening of the application, displaying the operating state.

In one or more exemplary methods, the wound dressing system system comprises a server device. In one or more exemplary methods, communicating 303 the operating state of the wound dressing comprises communicating 303c the operating state of the wound dressing to the server device. For example, communication the operating state of the wound dressing to the server device may comprise transmitting a message comprising the operating state to the server device from the accessory device.

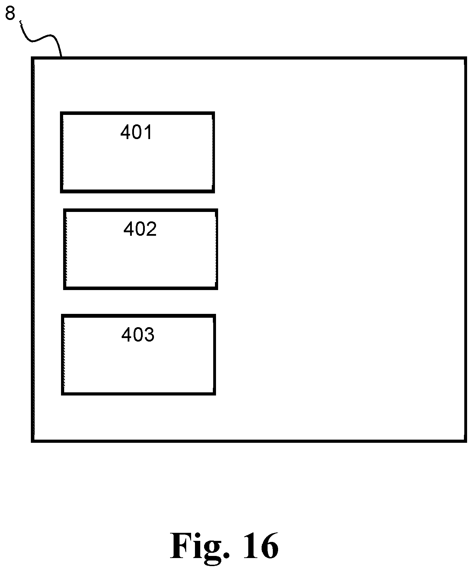

Fig. 16 is a block diagram illustrating an exemplary accessory device 8 according to the present disclosure. The accessory device 8 forms part of a wound dressing system and is capable of supporting the monitoring of the operating state of a wound dressing to be placed on a user' s skin. The accessory device 8 comprises a memory 401; a processor 402 coupled to the memory 401; and an interface 403, coupled to the processor 402.

Peripheral devices 401, 403 can be operatively and communicably coupled to the processor 402 via a bus for communicating data. The processor 402 can be a central processing unit (CPU), but other suitable microprocessors are also contemplated.

The interface 403 is configured to communicate with one or more devices of the wound dressing system. The one or more devices comprises a monitor device, and/or a wound dressing configured to be placed on a skin surface of a user or on any additional seals. The interface 403 is configured to obtain monitor data from the one or more devices, such as to receive or retrieve the monitor data from the one or more devices. The monitor data may be indicative of a condition of the wound dressing, such as a condition of the absorbent core layer of the wound dressing.

The processor 402 may be configured to determine an operating state of the wound dressing based on the monitor data obtained. The operating state may be indicative of a degree of wetting of the absorbent core layer of the wound dressing. The operating state may be indicative of a wetting pattern and/or wetting pattern type of a distal surface of the absorbent core layer of the wound dressing. The operating state may be indicative of absorbent performance of the wound dressing. The processor 402 may be configured to determine the operating state by determining a wetting pattern type. The wetting pattern type may be based on parameter data of the monitor data and/or one or more, such as all, of first monitor data, second monitor data, and third monitor data, such as first parameter data, second parameter data and the third parameter data. The wetting pattern type may be based on fourth parameter data. For example, the absorbent performance of the wound dressing is indicative of or comprises absorbent failure and/or saturation of the absorbent core layer and/or leakage risk of the wound dressing and/or indicative of the risk of skin damage or undesired healing conditions of the wound dressing system.

In one or more exemplary accessory devices, determination of a wetting pattern type may comprise selecting a wetting pattern type from a set of predefined wetting pattern types. The set of predefined wetting pattern types may comprise a number M of wetting pattern types, such as at least three wetting pattern types, at least four wetting pattern types, at least five wetting pattern types. The number M of wetting pattern types may be in the range from 4 to 100, such as from 5 to 20.

The memory 401 may be configured to store the monitor data and/or the operating state.

The interface 403 is configured to communicate the operating state of the wound dressing to the user via the interface, such as an audio interface, a visual interface, and/or a transceiver module. In one or more accessory devices, the interface 403 is configured to communicate the operating state of the wound dressing by displaying, on a visual interface (e.g. a display) of the accessory device, a user interface comprising a user interface object representative of the operating state, such as a first user interface object representative of a first operating state, a second user interface object representative of a second operating state, and/or a third user interface object representative of a third operating state. The user interface object may be representative of a determined wetting degree and/or wetting pattern and/or wetting pattern type, such as a first wetting pattern type, a second wetting pattern type, and/or a third wetting pattern type. In one or more accessory devices, the user interface object representative of the operating state comprises one or more of a first user interface object, and a second user interface object, wherein the first and/or second user interface objects are indicative of one or more of a first operating state, a second operating state and a third operating state. For example, the user interface object may comprise one or more visual indicators representative of the operating state, such as a first visual indicator, a second visual indicator, and/or a third visual indicator. For example, the user interface object may comprise one or more visual indicators representative of the determined wetting pattern and/or wetting pattern type, so as to mirror or represent the wetting pattern that is reflected by the obtained monitor data.

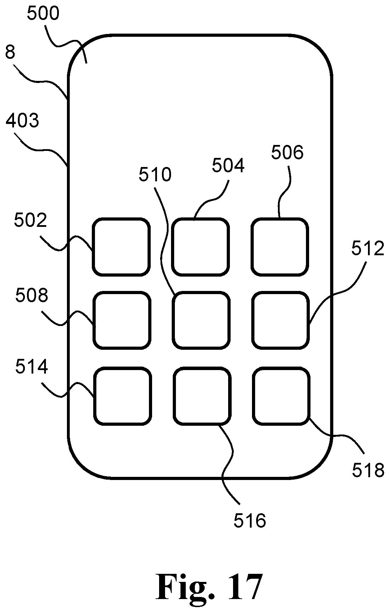

Figs. 17-18 illustrate exemplary user interfaces for communicating the operating state according to the present disclosure. Fig. 17 shows an exemplary user interface 500 for communicating the operating state of a wound dressing via accessory device 8, such as via a visual interface of accessory device interface 403. The user interface 500 comprises a plurality of visual indicators/user interface objects 502, 504, 506, 508, 510, 512, 514, 516, 518. Each visual indicator/user interface object 502, 504, 506, 508, 510, 512, 514, 516, 518 is representative of a condition of a sensor point or a group of sensor points of the wound dressing. For example, each visual indicator/user interface object 502, 504, 506, 508, 510, 512, 514, 516, 518 may have a first representation (e.g. first color and/or first shape) when no wetting of the sensor point is detected (high resistance of sensor point) and/or a second representation (e.g. second color and/or second shape) when wetting of the sensor point is detected (low resistance of sensor point). A first representation, such as a first color and/or a first shape, and/or a second representation, such as a second color and/or a second shape, of a visual indicator/user interface object may be based on monitor data obtained based on a plurality of sensor points. For example, a first representation of visual indicator/user interface object may be indicative of the number of wetted sensor points of a group of sensor points associated with the visual indicator/user interface object. In one or more exemplary user interfaces of the present disclosure, the number of sensor points associated with a visual indicator/user interface object of the user interface may be in the range from 1 to 100. A plurality of sensor points associated with a visual indicator/user interface object allows for a quantification of wetting. Thus, a visual indicator/user interface object may be representative of a quantification of wetting of a group of sensor points. The number of user interface objects of the user interface 500 may be less than the number of sensor points in the wound dressing. The number of user interface elements may be larger than 5, such as larger than 8.

It may be seen that the user interface is representative of a wound dressing where a user interface element is representative of a sensor point and/or define an area, zone, or sensing zone of the wound dressing. Thus, each user interface element 502, 504, 506, 508, 510, 512, 514, 516, 518 may be representative of a corresponding sensor point, e.g. sensor points SP_1_1, SP_1_2, SP_1_3, SP_2_1, SP_2_2, SP_2_3, SP_3_1, SP_3_2, SP_3_3, respectively.

The operating state of the wound dressing is optionally based on the determined wetting pattern type of the wound dressing using electrodes forming sensor points arranged in respective zones of the wound dressing. It may be seen that the user interface elements 502, 504, 506, 508, 510, 512, 514, 516, 518 are indicative of the monitor data, such as of electrical properties measured by the plurality of sensor points. The first user interface element 502 may be indicative of the operating state on first sensing zone (and/or a first zone) of the absorbent core layer/wound dressing. The second user interface element 504 may be indicative of the operating state on the second sensing zone (and/or a second zone) of the absorbent core layer/wound dressing. The third user interface element 506 may be indicative of the operating state on the third sensing zone (and/or a third zone) of the absorbent core layer/wound dressing.

The accessory device is configured to change dynamically the visual appearance of any of the visual indicators/user interface elements 502, 504, 506, 508, 510, 512, 514, 516, 518 based on the operating state of the wound dressing (i.e. so that the visual indicator reflects the current operating state of the wound dressing as determined by the accessory device). The visual appearance of a visual indicator, such as one or more of visual indicators 502, 504, 506, 508, 510, 512, 514, 516, 518, may be configured, by the accessory device, to change dynamically in terms of colour, shape, form, contrast, brightness, animation and/or blurring to indicate the operating state, e.g. to indicate the determined wetting pattern type and/or wetting degree. For example, the severity of the operating state may be displayed, by the accessory device, by varying in colour: e.g. blue, yellow, red for indicating low, medium, high severity respectively. For example, the severity of the operating state may be displayed, by the accessory device, by varying shades within a colour from a lighter shade to a darker shade for indicating low to high severity respectively. For example, the severity of the operating state may be displayed, by the accessory device, by varying a magnitude of the visual effect (e.g., less blurring, sharper contrast) as severity increases. In one or more exemplary accessory devices, a user interface element 502, 504, 506, 508, 510, 512, 514, 516, 518 displays a first color, such as green, if parameter data or wetting pattern type of the corresponding sensor point is indicative of no wetting of the corresponding sensor point on the distal surface of the absorbent core layer. In one or more exemplary accessory devices, a user interface element 502, 504, 506, 508, 510, 512, 514, 516, 518 displays a second color, such as red, if parameter data or wetting pattern type of the corresponding sensor point is indicative of wetting of the corresponding sensor point on the distal surface of the absorbent core layer.

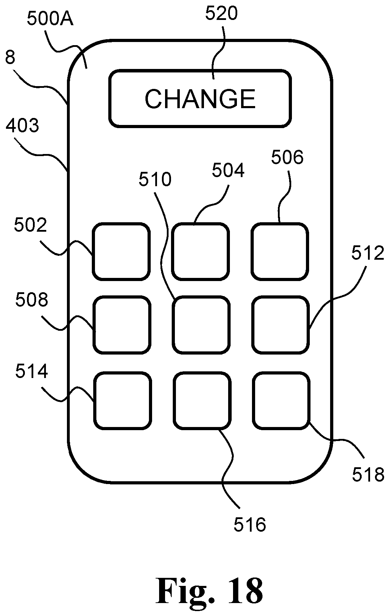

Fig. 18 shows an exemplary user interface 520A including a user interface element 520 indicative of a degree of wetting of the absorbent core layer/wound dressing. In one or more exemplary accessory devices, the user interface element 520 displays a warning color, such as red, and/or a warning text, such as "CHANGE", as shown, if a change criterion is satisfied. The change criterion may be satisfied if the number of parameters p_1_1, p_2_1, ..., p_N_1 having resistances less than a first threshold (i.e. the number of wetted sensor points) is larger than e.g. 0.9N, where N is the number of sensor points of the wound dressing. The use of a user interface element 520 indicative of a degree of wetting may as illustrated be combined with user interface elements 502, 504, 506, 508, 510, 512, 514, 516, 518 as described above.

The user interface element 520 may display a first color (corresponding to first operating state of wound dressing as indicated by monitor data), such as dark green, and/or a first text, such as "Low" or "Good", if the absorbent core layer is wetted to a first degree on the distal surface of the absorbent core layer, such as if a first common parameter being the number of parameters p_1_1, p_2_1, ..., p_N_1 having resistances less than a first threshold is in a first range, e.g. from 0.25N to 0.5N.

The user interface element 520 may display a second color (corresponding to second operating state of wound dressing as indicated by monitor data), such as yellow, and/or a second text, such as "Medium" or "Watch", if the absorbent core layer is wetted to a second degree on the distal surface of the absorbent core layer, such as if a first common parameter being the number of parameters p_1_1, p_2_1, ..., p_N_1 having resistances less than a first threshold is in a first range, e.g. from 0.5N to 0.75N.

The accessory device may be configured to provide the user interfaces, such as user interfaces 500, 500A of Fig. 17-18 in a user application running on the processor. The user application may be configured to communicate the operating state via the interface. The user application may be a dedicated wound dressing application that assist the user in monitoring the internal operating state of the wound dressing, and thereby reduce the likelihood of undesired healing conditions or severe leakage reaching out to clothing of the user.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary", "quaternary", "quinary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering. Furthermore, the labelling of a first element does not imply the presence of a second element and vice versa.

### List of references

1 wound dressing system
2, 2A wound dressing
4 monitor device
6 accessory device
8 server device
10 network
100 monitor device housing
101 processor 101
102 first interface (wound dressing interface)
104 second interface (accessory interface)
106 memory
110 first terminal
112 second terminal
114 third terminal
116 fourth terminal
118 fifth terminal
119 sixth terminal
120 coupling part
121 power unit
122 antenna
124 wireless transceiver
126 loudspeaker
128 haptic feedback element
140 sensor unit
150 first adhesive layer
150A proximal surface of the first adhesive layer
152 perforations of first adhesive layer
154 absorbing region
202 absorbent core layer
202A proximal surface of absorbent core layer
202B distal surface of absorbent core layer
204, 204A, 204B electrode assembly
206 top layer
207, 207A monitor interface
208 first support layer
208A proximal surface of first support layer
208B distal surface of first support layer
210 first set of first electrodes
210A, 210B, 210C, 210D, 210E, 210F, 210G, 210G, 210I first electrode
212 second set of second electrodes
212A, 212B, 212C second electrode
214 sensor point
215 connection parts of plurality of electrodes
216 first masking layer
218 first sensing part of first electrode
220 second sensing part of second electrode
222 sensor point opening of first masking layer
224 second support layer
224A proximal surface of second support layer
224B distal surface of second support layer
226 spacing layer
250 sensor point opening of first support layer
252 connection point/part of first electrode
254 sensor point opening of second support layer
256 connection point/part of second electrode
270, 270A second masking layer
272 sensor point openings
300 method for communicating an operating state of a wound dressing
301 obtaining monitor data from the one or more devices
301a obtaining parameter data indicative of resistances measured on a distal surface of the absorbent core layer of the wound dressing
301b obtaining data representative of the measurement of the electrical properties of the absorbent core layer of the wound dressing
301c obtaining wound data and/or parameter data
302 determining an operating state of the wound dressing based on the monitor data
302a determining a wetting pattern type based on the monitor data
302aa comparing first parameter data and second parameter data
302ab identifying a wetting pattern type based on the comparison
302aaa determining whether the first parameter data and/or the second parameter data satisfy one or more criteria
302aab identifying a wetting pattern type based on the determination
302b determining a degree of wetting of the absorbent core layer
302c deriving the operating state
303 communicating the operating state of the wound dressing via the interface
303a displaying, on a visual interface of the accessory device, a user interface comprising a user interface object representative of the operating state
303b notifying the user via the interface
303c communicating the operating state of the wound dressing to the server device
401 accessory device memory
402 accessory device processor
403 accessory device interface
500, 500A user interface
502 first user interface element
504 second user interface element
506 third user interface element
508 fourth user interface element
510 fifth user interface element
512 sixth user interface element
514 seventh user interface element
516 eighth user interface element
518 ninth user interface element
520 user interface element indicative of degree of wetting.

## Claims

1. A method (300), performed in an accessory device (6), for communicating an operating state of a wound dressing (2, 2A) comprising an absorbent core layer (202), wherein the accessory device (6) comprises an interface (403) configured to communicate with one or more devices of a wound dressing system (1), the one or more devices comprising a monitor device (4) connected to a wound dressing (2, 2A), wherein the wound dressing is placed on a skin surface of a user, the method comprising:
obtaining (301) monitor data from the monitor device, the monitor data being indicative of a condition of the wound dressing;
determining (302) an operating state of the wound dressing based on the monitor data, wherein the operating state is indicative of a degree of wetting of the absorbent core layer of the wound dressing and indicative of a wetting pattern of the absorbent core layer; and
communicating (303) the operating state of the wound dressing via the interface.

2. Method according to claim 1, wherein the monitor data are indicative of wetting of a distal surface (202B) of the absorbent core layer.

3. Method according to any of claims 1-2, wherein the wound dressing comprises an electrode assembly (204, 204A, 204B) comprising a plurality of sensor points (214) configured to measure electrical properties of the absorbent core layer and wherein obtaining monitor data comprises obtaining (301b) data representative of the measurement of the electrical properties of the absorbent core layer.

4. Method according to any of the preceding claims, wherein obtaining the monitor data comprises obtaining the monitor data comprising wound data and/or parameter data based on the wound data.

5. Method according to claim 4, wherein the wound data and/or parameter data are indicative of resistance between electrodes, capacitance and/or inductance between electrodes and/or any change thereof.

6. Method according to any of claims 1-5, wherein the monitor data comprises localized monitor data with respect to a location and/or zone on the absorbent core layer.

7. Method according to any of the preceding claims, wherein determining the operating state of the wound dressing based on the monitor data comprises determining a wetting pattern of the absorbent core layer and determining a wetting pattern type of the wetting pattern from a plurality of wetting pattern types.

8. Method according to claim 7, wherein determining (302) a wetting pattern type based on the monitor data comprises comparing (302a) first parameter data and second parameter data and identifying (302ab) a wetting pattern type based on the comparison.

9. Method according to claim 8, wherein comparing the first parameter data and/or the second parameter data comprises determining (302aaa) whether the first parameter data and/or the second parameter data satisfy one or more criteria and identifying a wetting pattern type based on the determination.

10. Method according to any of claims 7-9, wherein determining the operating state of the wound dressing based on the monitor data comprises deriving (302c) the operating state based on one or more wetting pattern types.

11. Method according to any of the preceding claims, wherein the interface comprises an audio interface, a visual interface, and/or a transceiver module.

12. Method according to any of the preceding claims, wherein communicating the operating state of the wound dressing comprises displaying (303a), on a visual interface of the accessory device, a user interface (500, 500A) comprising a user interface object representative of the operating state.

13. Method according to claim 12, wherein the user interface object representative of the operating state comprises one or more of a first user interface object, and a second user interface object, wherein the first and/or second user interface objects are indicative of one or more of a first operating state, a second operating state and a third operating state.

14. Method according to any of the preceding claims, wherein communicating the operating state of the wound dressing comprises notifying (303b) the user via the interface.

15. Method according to any of the preceding claims, wherein the wound dressing system comprises a server device (8), and wherein communicating the operating state of the wound dressing comprises communicating (303c) the operating state of the wound dressing to the server device.

16. An accessory device (6), wherein the accessory device forms part of a wound dressing system (1), the accessory device (6) comprising:
a memory (401);
a processor (402) coupled to the memory; and
an interface (403), coupled to the processor, configured to communicate with one or more devices of the wound dressing system, the one or more devices comprising a monitor device (4) connectable to a wound dressing (2, 2A) configured to be placed on a skin surface of a user;
wherein the interface (403) is configured to obtain monitor data from the monitor device, the monitor data being indicative of a condition of the wound dressing;
wherein the processor (402) is configured to determine an operating state of the wound dressing based on the monitor data, wherein the operating state is indicative of a degree of wetting of the absorbent core layer of the wound dressing and indicative of a wetting pattern of the absorbent core layer;
wherein the memory (401) is configured to store the monitor data and/or the operating state; and
wherein the accessory device is configured to communicate the operating state of the wound dressing to the user via the interface (403).

17. A wound dressing system (1) comprising a wound dressing (2, 2A), an accessory device (6) and a monitor device (4), the wound dressing (2, 2A) comprising an absorbent core layer (202), wherein the accessory device (6) is configured to perform any of the methods according to any of claims 1-15.

18. A computer readable storage medium storing one or more programs, the one or more programs comprising instructions, which when executed by an accessory device (6) with an interface (403), a memory (401) and a processor (402) cause the accessory device (6) to perform any of the methods of claims 1-15.

## Patentansprüche

1. Verfahren (300), das in einer Zubehörvorrichtung (6) zum Übermitteln eines Betriebszustands eines Wundverbands (2, 2A) durchgeführt wird, der eine absorbierende Kernschicht (202) umfasst, wobei die Zubehörvorrichtung (6) eine Schnittstelle (403) umfasst, die dafür ausgelegt ist, mit einer oder mehreren Vorrichtungen eines Wundverbandsystems (1) zu kommunizieren, wobei die eine oder die mehreren Vorrichtungen eine Überwachungsvorrichtung (4) umfassen, die mit einem Wundverband (2, 2A) verbunden ist, wobei der Wundverband auf einer Hautoberfläche eines Benutzers platziert wird, wobei das Verfahren folgende Vorgänge umfasst:
Erhalten (301) von Überwachungsdaten von der Überwachungsvorrichtung, wobei die Überwachungsdaten einen Zustand des Wundverbands angeben;
Bestimmen (302) eines Betriebszustands des Wundverbands auf der Grundlage der Überwachungsdaten, wobei der Betriebszustand einen Grad von Benetzung der absorbierenden Kernschicht des Wundverbands und ein Benetzungsmuster der absorbierenden Kernschicht angibt; und
Übermitteln (303) des Betriebszustands des Wundverbands über die Schnittstelle.

2. Verfahren nach Anspruch 1, wobei die Überwachungsdaten die Benetzung einer distalen Oberfläche (202B) der absorbierenden Kernschicht angeben.

3. Verfahren nach einem der Ansprüche 1 bis 2, wobei der Wundverband eine Elektrodenanordnung (204, 204A, 204B) umfasst, die eine Vielzahl von Sensorpunkten (214) umfasst, die dafür ausgelegt sind, elektrische Eigenschaften der absorbierenden Kernschicht zu messen, und wobei das Erhalten von Überwachungsdaten das Erhalten (301b) von Daten umfasst, die für die Messung der elektrischen Eigenschaften der absorbierenden Kernschicht repräsentativ sind.

4. Verfahren nach einem der vorhergehenden Ansprüche, wobei das Erhalten der Überwachungsdaten das Erhalten der Überwachungsdaten umfasst, die Wunddaten und/oder auf den Wunddaten basierende Parameterdaten umfassen.

5. Verfahren nach Anspruch 4, wobei die Wunddaten und/oder die Parameterdaten Widerstand zwischen Elektroden, Kapazität und/oder Induktivität zwischen Elektroden und/oder jegliche Änderung derselben angeben.

6. Verfahren nach einem der Ansprüche 1 bis 5, wobei die Überwachungsdaten lokalisierte Überwachungsdaten in Bezug auf eine Stelle und/oder eine Zone auf der absorbierenden Kernschicht umfassen.

7. Verfahren nach einem der vorhergehenden Ansprüche, wobei das Bestimmen des Betriebszustands des Wundverbands auf der Grundlage der Überwachungsdaten das Bestimmen eines Benetzungsmusters der absorbierenden Kernschicht und das Bestimmen einer Benetzungsmusterart des Benetzungsmusters aus einer Vielzahl von Benetzungsmusterarten umfasst.

8. Verfahren nach Anspruch 7, wobei das Bestimmen (302) einer Benetzungsmusterart auf der Grundlage der Überwachungsdaten das Vergleichen (302a) erster Parameterdaten und zweiter Parameterdaten und das Identifizieren (302ab) einer Benetzungsmusterart auf der Grundlage des Vergleichs umfasst.

9. Verfahren nach Anspruch 8, wobei das Vergleichen der ersten Parameterdaten und/oder der zweiten Parameterdaten das Bestimmen (302aaa), ob die ersten Parameterdaten und/oder die zweiten Parameterdaten ein oder mehrere Kriterien erfüllen, und das Identifizieren einer Benetzungsmusterart auf der Grundlage der Bestimmung umfasst.

10. Verfahren nach einem der Ansprüche 7 bis 9, wobei das Bestimmen des Betriebszustands des Wundverbands auf der Grundlage der Überwachungsdaten das Ableiten (302c) des Betriebszustands auf der Grundlage einer oder mehrerer Benetzungsmusterarten umfasst.

11. Verfahren nach einem der vorhergehenden Ansprüche, wobei die Schnittstelle eine Audioschnittstelle, eine visuelle Schnittstelle und/oder ein Sender-Empfänger-Modul umfasst.

12. Verfahren nach einem der vorhergehenden Ansprüche, wobei das Übermitteln des Betriebszustands des Wundverbands das Anzeigen (303a), auf einer visuellen Schnittstelle der Zubehörvorrichtung, einer Benutzerschnittstelle (500, 500A) umfasst, die ein für den Betriebszustand repräsentatives Benutzerschnittstellenobjekt umfasst.

13. Verfahren nach Anspruch 12, wobei das für den Betriebszustand repräsentative Benutzerschnittstellenobjekt eines oder mehrere von einem ersten Benutzerschnittstellenobjekt und einem zweiten Benutzerschnittstellenobjekt umfasst, wobei das erste und/oder das zweite Benutzerschnittstellenobjekt einen oder mehrere von einem ersten Betriebszustand, einem zweiten Betriebszustand und einem dritten Betriebszustand angeben.

14. Verfahren nach einem der vorhergehenden Ansprüche, wobei das Übermitteln des Betriebszustands des Wundverbands das Benachrichtigen (303b) des Benutzers über die Schnittstelle umfasst.

15. Verfahren nach einem der vorhergehenden Ansprüche, wobei das Wundverbandsystem eine Servervorrichtung (8) umfasst und wobei das Übermitteln des Betriebszustands des Wundverbands das Übermitteln (303c) des Betriebszustands des Wundverbands an die Servervorrichtung umfasst.

16. Zubehörvorrichtung (6), wobei die Zubehörvorrichtung einen Teil eines Wundverbandsystems (1) bildet, wobei die Zubehörvorrichtung (6) umfasst:
einen Speicher (401);
einen Prozessor (402), der mit dem Speicher gekoppelt ist; und
eine mit dem Prozessor gekoppelte Schnittstelle (403), die dafür ausgelegt ist, mit einer oder mehreren Vorrichtungen des Wundverbandsystems zu kommunizieren, wobei die eine oder die mehreren Vorrichtungen eine Überwachungsvorrichtung (4) umfassen, die mit einem Wundverband (2, 2A) verbunden werden kann, der dafür ausgelegt ist, auf einer Hautoberfläche eines Benutzers platziert zu werden;
wobei die Schnittstelle (403) dafür ausgelegt ist, Überwachungsdaten von der Überwachungsvorrichtung zu erhalten, wobei die Überwachungsdaten einen Zustand des Wundverbands angeben;
wobei der Prozessor (402) dafür ausgelegt ist, einen Betriebszustand des Wundverbands auf der Grundlage der Überwachungsdaten zu bestimmen, wobei der Betriebszustand einen Grad von Benetzung der absorbierenden Kernschicht des Wundverbands angibt und ein Benetzungsmuster der absorbierenden Kernschicht angibt;
wobei der Speicher (401) zum Speichern der Überwachungsdaten und/oder des Betriebszustands ausgelegt ist; und
wobei die Zubehörvorrichtung dafür ausgelegt ist, dem Benutzer über die Schnittstelle (403) den Betriebszustand des Wundverbands zu übermitteln.

17. Wundverbandsystem (1), umfassend einen Wundverband (2, 2A), eine Zubehörvorrichtung (6) und eine Überwachungsvorrichtung (4), wobei der Wundverband (2, 2A) eine absorbierende Kernschicht (202) umfasst, wobei die Zubehörvorrichtung (6) dafür ausgelegt ist, eines der Verfahren nach einem der Ansprüche 1 bis 15 durchzuführen.

18. Computerlesbares Speichermedium, auf dem ein oder mehrere Programme gespeichert sind, wobei das eine oder die mehreren Programme Anweisungen umfassen, die bei ihrer Ausführung durch eine Zubehörvorrichtung (6) mit einer Schnittstelle (403), einem Speicher (401) und einem Prozessor (402) die Zubehörvorrichtung (6) veranlassen, eines der Verfahren nach den Ansprüchen 1 bis 15 durchzuführen.

## Revendications

1. Procédé (300), réalisé dans un dispositif accessoire (6), pour communiquer un état de fonctionnement d'un pansement (2, 2A) comportant une couche centrale absorbante (202), le dispositif accessoire (6) comportant une interface (403) configurée pour communiquer avec un ou plusieurs dispositifs d'un système (1) de pansement, le ou les dispositifs comportant un dispositif (4) de surveillance relié à un pansement (2, 2A), le pansement étant placé sur une surface de peau d'un utilisateur, le procédé comportant les étapes consistant à :
obtenir (301) des données de surveillance à partir du dispositif de surveillance, les données de surveillance étant indicatives d'une condition du pansement ;
déterminer (302) un état de fonctionnement du pansement d'après les données de surveillance, l'état de fonctionnement étant indicatif d'un degré de mouillage de la couche centrale absorbante du pansement et indicatif d'un diagramme de mouillage de la couche centrale absorbante ; et
communiquer (303) l'état de fonctionnement du pansement via l'interface.

2. Procédé selon la revendication 1, les données de surveillance étant indicatives d'un mouillage d'une surface distale (202B) de la couche centrale absorbante.

3. Procédé selon l'une quelconque des revendications 1 à 2, le pansement comportant un ensemble (204, 204A, 204B) d'électrodes comportant une pluralité de points (214) de capteurs configurés pour mesurer des propriétés électriques de la couche centrale absorbante et l'obtention de données de surveillance comportant l'obtention (301b) de données représentatives de la mesure des propriétés électriques de la couche centrale absorbante.

4. Procédé selon l'une quelconque des revendications précédentes, l'obtention des données de surveillance comportant l'obtention des données de surveillance comprenant des données de plaie et/ou des données de paramètres basées sur les données de plaie.

5. Procédé selon la revendication 4, les données de plaie et/ou les données de paramètres étant indicatives d'une résistance entre des électrodes, d'une capacitance et/ou d'une inductance entre des électrodes et/ou d'un quelconque changement de celles-ci.

6. Procédé selon l'une quelconque des revendications 1 à 5, les données de surveillance comportant des données de surveillance localisées par rapport à un emplacement et/ou à une zone sur la couche centrale absorbante.

7. Procédé selon l'une quelconque des revendications précédentes, la détermination de l'état de fonctionnement du pansement d'après les données de surveillance comportant la détermination d'un diagramme de mouillage de la couche centrale absorbante et la détermination d'un type de diagramme de mouillage du diagramme de mouillage parmi une pluralité de types de diagramme de mouillage.

8. Procédé selon la revendication 7, la détermination (302) d'un type de diagramme de mouillage d'après les données de surveillance comportant la comparaison (302a) de premières données de paramètres et de deuxièmes données de paramètres et l'identification (302ab) d'un type de diagramme de mouillage d'après la comparaison.

9. Procédé selon la revendication 8, la comparaison des premières données de paramètres et/ou des deuxièmes données de paramètres comportant le fait de déterminer (302aaa) si les premières données de paramètres et/ou les deuxièmes données de paramètres satisfont un ou plusieurs critères et d'identifier un type de diagramme de mouillage d'après la détermination.

10. Procédé selon l'une quelconque des revendications 7 à 9, la détermination de l'état de fonctionnement du pansement d'après les données de surveillance comportant la déduction (302c) de l'état de fonctionnement d'après un ou plusieurs types de diagramme de mouillage.

11. Procédé selon l'une quelconque des revendications précédentes, l'interface comportant une interface audio, une interface visuelle, et/ou un module émetteur-récepteur.

12. Procédé selon l'une quelconque des revendications précédentes, la communication de l'état de fonctionnement du pansement comportant l'affichage (303a), sur une interface visuelle du dispositif accessoire, d'une interface (500, 500A) d'utilisateur comportant un objet d'interface d'utilisateur représentatif de l'état de fonctionnement.

13. Procédé selon la revendication 12, l'objet d'interface d'utilisateur représentatif de l'état de fonctionnement comportant un ou plusieurs objets parmi un premier objet d'interface d'utilisateur et un deuxième objet d'interface d'utilisateur, les premier et/ou deuxième objets d'interface d'utilisateur étant indicatifs d'un ou plusieurs états parmi un premier état de fonctionnement, un deuxième état de fonctionnement et un troisième état de fonctionnement.

14. Procédé selon l'une quelconque des revendications précédentes, la communication de l'état de fonctionnement du pansement comportant la notification (303b) de l'utilisateur via l'interface.

15. Procédé selon l'une quelconque des revendications précédentes, le système de pansement comportant un dispositif serveur (8), et la communication de l'état de fonctionnement du pansement comportant la communication (303c) de l'état de fonctionnement du pansement au dispositif serveur.

16. Dispositif accessoire (6), le dispositif accessoire faisant partie d'un système (1) de pansement, le dispositif accessoire (6) comportant :
une mémoire (401) ;
un processeur (402) couplé à la mémoire ; et
une interface (403), couplée au processeur, configurée pour communiquer avec un ou plusieurs dispositifs du système de pansement, le ou les dispositifs comportant un dispositif (4) de surveillance susceptible d'être relié à un pansement (2, 2A) configuré pour être placé sur une surface de peau d'un utilisateur ;
l'interface (403) étant configurée pour obtenir des données de surveillance à partir du dispositif de surveillance, les données de surveillance étant indicatives d'une condition du pansement ;
le processeur (402) étant configuré pour déterminer un état de fonctionnement du pansement d'après les données de surveillance, l'état de fonctionnement étant indicatif d'un degré de mouillage de la couche centrale absorbante du pansement et indicatif d'un diagramme de mouillage de la couche centrale absorbante ;
la mémoire (401) étant configurée pour stocker les données de surveillance et/ou l'état de fonctionnement ; et
le dispositif accessoire étant configuré pour communiquer l'état de fonctionnement du pansement à l'utilisateur via l'interface (403).

17. Système (1) de pansement comportant un pansement (2, 2A), un dispositif accessoire (6) et un dispositif (4) de surveillance, le pansement (2, 2A) comportant une couche centrale absorbante (202), le dispositif accessoire (6) étant configuré pour réaliser l'un quelconque des procédés selon l'une quelconque des revendications 1 à 15.

18. Support de stockage lisible par ordinateur conservant un ou plusieurs programmes, le ou les programmes comportant des instructions qui, lorsqu'elles sont exécutées par un dispositif accessoire (6) doté d'une interface (403), d'une mémoire (401) et d'un processeur (402), amènent le dispositif accessoire (6) à réaliser l'un quelconque des procédés des revendications 1 à 15.
